(19) Europäisches Patentamt
European Patent Office
Office européen des brevets

(11) **EP 1 259 608 B1**

(12) **EUROPEAN PATENT SPECIFICATION**

(45) Date of publication and mention
of the grant of the patent:
**12.08.2009 Bulletin 2009/33**

(21) Application number: **01910621.0**

(22) Date of filing: **13.02.2001**

(51) Int Cl.:
*C12N 15/12* (2006.01)       *C07K 14/435* (2006.01)
*C12N 15/62* (2006.01)       *G01N 33/52* (2006.01)
*A61K 31/00* (2006.01)       *A61K 33/00* (2006.01)

(86) International application number:
**PCT/US2001/004625**

(87) International publication number:
**WO 2001/062919 (30.08.2001 Gazette 2001/35)**

(54) **MODIFIED FLUORESCENT PROTEINS**

VERÄNDERTE, FLUORESZIERENDE PROTEINE

PROTEINES FLUORESCENTES MODIFIEES

(84) Designated Contracting States:
**AT BE CH CY DE DK ES FI FR GB GR IE IT LI LU
MC NL PT SE TR**

(30) Priority: **23.02.2000 US 184732 P**

(43) Date of publication of application:
**27.11.2002 Bulletin 2002/48**

(73) Proprietor: **Life Technologies Corporation
Carlsbad, CA 92008 (US)**

(72) Inventors:
• **NELSON, David
San Diego, CA 92121 (US)**
• **ZAMAIRA, Elize
San Diego, CA 92131 (US)**
• **TSIEN, Roger
La Jolla, CA 92037 (US)**

(74) Representative: **Couchman, Jonathan Hugh et al
Harrison Goddard Foote
Belgrave Hall
Belgrave Street
Leeds
LS2 8DD (GB)**

(56) References cited:
**WO-A-00/34326**       **WO-A-01/27150**

• **MATZ M V ET AL: "FLUORESCENT PROTEINS
FROM NONBIOLUMINESCENT ANTHOZOA
SPECIES" NATURE BIOTECHNOLOGY,NATURE
PUBLISHING,US, vol. 17, October 1999 (1999-10),
pages 969-973, XP000882891 ISSN: 1087-0156**
• **FRADKOV A F ET AL: "NOVEL FLUORESCENT
PROTEIN FROM DISCOSOMA CORAL AND ITS
MUTANTS POSSESSES A UNIQUE FAR-RED
FLUORESCENCE" FEBS LETTERS,ELSEVIER
SCIENCE PUBLISHERS, AMSTERDAM,NL, vol.
479, 18 August 2000 (2000-08-18), pages 127-130,
XP000941962 ISSN: 0014-5793**

Remarks:
The file contains technical information submitted after
the application was filed and not included in this
specification

EP 1 259 608 B1

SEQUENCE ID. LISTING

    (1) GENERAL INFORMATION:

        (iii) NUMBER OF SEQUENCES: 10

    (2) INFORMATION FOR SEQ. ID. NO.: 1:

        (i) SEQUENCE CHARACTERISTICS:

            (A) LENGTH: 690 base pairs
            (B) TYPE: nucleic acid
            (C) STRANDEDNESS: double

(D) TOPOLOGY: linear

(ii) MOLECULE TYPE: cDNA
(ix) FEATURE:

(A) NAME / KEY: Coding Sequence
(B) LOCATION: 1....690

(xi) SEQUENCE DESCRIPTION: SEQ. ID. NO.1:


ATG GCT CTT TCA AAC AAG TTT ATC GGA GAT GAC ATG AAA ATG ACC TAC CAT

ATG GAT GGC TGT GTC AAT GGG CAT TAC TTT ACC GTC AAA GGT GAA GGC AAC

GGG AAG CCA TAC GAA GGG ACG CAG ACT TCG ACT TTT AAA GTC ACC ATG GCC

AAC GGT GGG CCC CTT GCA TTC TCC TTT GAC ATA CTA TCT ACA GTG TTC AAA

TAT GGA AAT CGA TGC TTT ACT GCG TAT CCT ACC AGT ATG CCC GAC TAT TTC

AAA CAA GCA TTT CCT GAC GGA ATG TCA TAT GAA AGG ACT TTT ACC TAT GAA

GAT GGA GGA GTT GCT ACA GCC AGT TGG GAA ATA AGC CTT AAA GGC AAC TGC

TTT GAG CAC AAA TCC ACG TTT CAT GGA GTG AAC TTT CCT GCT GAT GGA CCT

GTG ATG GCG AAG AAG ACA ACT GGT TGG GAC CCA TCT TTT GAG AAA ATG ACT

GTC TGC GAT GGA ATA TTG AAG GGT GAT GTC ACC GCG TTC CTC ATG CTG CAA

GGA GGT GGC AAT TAC AGA TGC CAA TTC CAC ACT TCT TAC AAG ACA AAA AAA

CCG GTG ACG ATG CCA CCA AAC CAT GTG GTG GAA CAT CGC ATT GCG AGG ACC

GAC CTT GAC AAA GGT GGC AAC AGT GTT CAG CTG ACG GAG CAC GCT GTT GCA

CAT ATA ACC TCT GTT GTC CCT TTC TGA


(2) INFORMATION FOR SEQ. ID. NO.2:

(i) SEQUENCE CHARACTERISTICS:

(A) LENGTH: 696 base pairs
(B) TYPE: nucleic acid
(C) STRANDEDNESS: double
(D) TOPOLOGY: linear

(ii) MOLECULE TYPE: cDNA
(ix) FEATURE:

(A) NAME / KEY: Coding Sequence
(B) LOCATION: 1....696

(xi) SEQUENCE DESCRIPTION: SEQ. ID. NO.2:

```
ATG GCT CAG TCA AAG CAC GGT CTA ACA AAA GAA ATG ACA ATG AAA TAC CGT

ATG GAA GGG TGC GTC GAT GGA CAT AAA TTT GTG ATC ACG GGA GAG GGC ATT

GGA TAT CCG TTC AAA GGG AAA CAG GCT ATT AAT CTG TGT GTG GTC GAA GGT

GGA CCA TTG CCA TTT GCC GAA GAC ATA TTG TCA GCT GCC TTT AAC TAC GGA

AAC AGG GTT TTC ACT GAA TAT CCT CAA GAC ATA GTT GAC TAT TTC AAG AAC

TCG TGT CCT GCT GGA TAT ACA TGG GAC AGG TCT TTT CTC TTT GAG GAT GGA

GCA GTT TGC ATA TGT AAT GCA GAT ATA ACA GTG AGT GTT GAA GAA AAC TGC

ATG TAT CAT GAG TCC AAA TTT TAT GGA GTG AAT TTT CCT GCT GAT GGA CCT

GTG ATG AAA AAG ATG ACA GAT AAC TGG GAG CCA TCC TGC GAG AAG ATC ATA

CCA GTA CCT AAG CAG GGG ATA TTG AAA GGG GAT GTC TCC ATG TAC CTC CTT

CTG AAG GAT GGT GGG CGT TTA CGG TGC CAA TTC GAC ACA GTT TAC AAA GCA

AAG TCT GTG CCA AGA AAG ATG CCG GAC TGG CAC TTC ATC AGC CAT AAG CTC

ACC CGT GAA GAC CGC AGC GAT GCT AAG AAT CAG AAA TGG CAT CTG ACA GAA

CAT GCT ATT GCA TCC GGA TCT GCA TTG CCC TGA
```

(2) INFORMATION FOR SEQ. ID. NO.3:

    (i) SEQUENCE CHARACTERISTICS:

        (A) LENGTH: 696 base pairs
        (B) TYPE: nucleic acid
        (C) STRANDEDNESS: double
        (D) TOPOLOGY: linear

    (ii) MOLECULE TYPE: cDNA
    (ix) FEATURE:

        (A) NAME / KEY: Coding Sequence
        (B) LOCATION: 1...696

    (xi) SEQUENCE DESCRIPTION: SEQ. ID. NO.3:

ATG GCT CAT TCA AAG CAC GGT CTA AAA GAA GAA ATG ACA ATG AAA TAC CAC

ATG GAA GGG TGC GTC AAC GGA CAT AAA TTT GTG ATC ACG GGC GAA GGC ATT

GGA TAT CCG TTC AAA GGG AAA CAG ACT ATT AAT CTG TGT GTG ATC GAA GGG

GGA CCA TTG CCA TTT TCC GAA GAC ATA TTG TCA GCT GGC TTT AAG TAC GGA

GAC AGG ATT TTC ACT GAA TAT CCT CAA GAC ATA GTA GAC TAT TTC AAG AAC

TCG TGT CCT GCT GGA TAT ACA TGG GGC AGG TCT TTT CTC TTT GAG GAT GGA

GCA GTC TGC ATA TGC AAT GTA GAT ATA ACA GTG AGT GTC AAA GAA AAC TGC

ATT TAT CAT AAG AGC ATA TTT AAT GGA ATG AAT TTT CCT GCT GAT GGA CCT

GTG ATG AAA AAG ATG ACA ACT AAC TGG GAA GCA TCC TGC GAG AAG ATC ATG

CCA  GTA CCT AAG CAG GGG ATA CTG AAA GGG GAT GTC TCC ATG TAC CTC CTT

CTG AAG GAT GGT GGG CGT TAC CGG TGC CAG TTC GAC ACA GTT TAC AAA GCA

AAG TCT GTG CCA AGT AAG ATG CCG GAG TGG CAC TTC ATC CAG CAT AAG CTC

CTC CGT GAA GAC CGC AGC GAT GCT AAG AAT CAG AAG TGG CAG CTG ACA GAG

CAT GCT ATT GCA TTC CCT TCT GCC TTG GCC TGA

(2) INFORMATION FOR SEQ. ID. NO.4:

    (i) SEQUENCE CHARACTERISTICS:

        (A) LENGTH: 699 base pairs
        (B) TYPE: nucleic acid
        (C) STRANDEDNESS: double
        (D) TOPOLOGY: linear

    (ii) MOLECULE TYPE: cDNA
    (ix) FEATURE:

        (A) NAME / KEY: Coding Sequence
        (B) LOCATION: 1...699

    (xi) SEQUENCE DESCRIPTION: SEQ. ID. NO.4:

```
ATG AGT TGT TCC AAG AGT GTG ATC AAG GAA GAA ATG TTG ATC GAT CTT CAT

CTG GAA GGA ACG TTC AAT GGG CAC TAC TTT GAA ATA AAA GGC AAA GGA AAA

GGA CAG CCT AAT GAA GGC ACC AAT ACC GTC ACG CTC GAG GTT ACC AAG GGT

GGA CCT CTG CCA TTT GGT TGG CAT ATT TTG TGC CCA CAA TTT CAG TAT GGA

AAC AAG GCA TTT GTC CAC CAC CCT GAC AAC ATA CAT GAT TAT CTA AAG CTG

TCA TTT CCG GAG GGA TAT ACA TGG GAA CGG TCC ATG CAC TTT GAA GAC GGT

GGC TTG TGT TGT ATC ACC AAT GAT ATC AGT TTG ACA GGC AAC TGT TTC TAC

TAC GAC ATC AAG TTC ACT GGC TTG AAC TTT CCT CCA AAT GGA CCC GTT GTG

CAG AAG AAG ACA ACT GGC TGG GAA CCG AGC ACT GAG CGT TTG TAT CCT CGT

GAT GGT GTG TTG ATA GGA GAC ATC CAT CAT GCT CTG ACA GTT GAA GGA GGT

GGT CAT TAC GCA TGT GAC ATT AAA ACT GTT TAC AGG GCC AAG AAG GCC GCC

TTG AAG ATG CCA GGG TAT CAC TAT GTT GAC ACC AAA CTG GTT ATA TGG AAC

AAC GAC AAA GAA TTC ATG AAA GTT GAG GAG CAT GAA ATC GCC GTT GCA CGC

CAC CAT CCG TTC TAT GAG CCA AAG AAG GAT AAG TAA
```

(2) INFORMATION FOR SEQ. ID. NO.5:

    (i) SEQUENCE CHARACTERISTICS:

        (A) LENGTH: 678 base pairs
        (B) TYPE: nucleic acid
        (C) STRANDEDNESS: double
        (D) TOPOLOGY: linear

    (ii) MOLECULE TYPE: cDNA
    (ix) FEATURE:

        (A) NAME / KEY: Coding Sequence
        (B) LOCATION: 1....678

    (xi) SEQUENCE DESCRIPTION: SEQ. ID. NO.5:

ATG AGG TCT TCC AAG AAT GTT ATC AAG GAG TTC ATG AGG TTT AAG GTT CGC
ATG

GAA GGA ACG GTC AAT GGG CAC GAG TTT GAA ATA GAA GGC GAA GGA GAG GGG
AGG

CCA TAC GAA GGC CAC AAT ACC GTA AAG CTT AAG GTA ACC AAG GGG GGA CCT
TTG

CCA TTT GCT TGG GAT ATT TTG TCA CCA CAA TTT CAG TAT GGA AGC AAG GTA
TAT

GTC AAG CAC CCT GCC GAC ATA CCA GAC TAT AAA AAG CTG TCA TTT CCT GAA
GGA

TTT AAA TGG GAA AGG GTC ATG AAC TTT GAA GAC GGT GGC GTC GTT ACT GTA
ACC

CAG GAT TCC AGT TTG CAG GAT GGC TGT TTC ATC TAC AAG GTC AAG TTC ATT
GGC

GTG AAC TTT CCT TCC GAT GGA CCT GTT ATG CAA AAG AAG ACA ATG GGC TGG
GAA

GCC AGC ACT GAG CGT TTG TAT CCT CGT GAT GGC GTG TTG AAA GGA GAG ATT
CAT

AAG GCT CTG AAG CTG AAA GAC GGT GGT CAT TAC CTA GTT GAA TTC AAA AGT
ATT

TAC ATG GCA AAG AAG CCT GTG CAG CTA CCA GGG TAC TAC TAT GTT GAC TCC
AAA

CTG GAT ATA ACA AGC CAC AAC GAA GAC TAT ACA ATC GTT GAG CAG TAT GAA
AGA

ACC GAG GGA CGC CAC CAT CTG TTC CTT TAA

(2) INFORMATION FOR SEQ. ID. NO.6:

    (i) SEQUENCE CHARACTERISTICS:

       (A) LENGTH: 801 base pairs
       (B) TYPE: nucleic acid
       (C) STRANDEDNESS: double
       (D) TOPOLOGY: linear

    (ii) MOLECULE TYPE: cDNA
    (ix) FEATURE:

       (A) NAME / KEY: Coding Sequence
       (B) LOCATION: 1......801

    (xi) SEQUENCE DESCRIPTION: SEQ. ID. NO.6:

```
ATG AAG TGT AAA TTT GTG TTC TGC CTG TCC TTC TTG GTC CTC GCC ATC ACA

AAC GCG AAC ATT TTT TTG AGA AAC GAG GCT GAC TTA GAA GAG AAG ACA TTG

AGA ATA CCA AAA GCT CTA ACC ACC ATG GGT GTG ATT AAA CCA GAC ATG AAG

ATT AAG CTG AAG ATG GAA GGA AAT GTA AAC GGG CAT GCT TTT GTG ATC GAA

GGA GAA GGA GAA GGA AAG CCT TAC GAT GGG ACA CAC ACT TTA AAC CTG GAA

GTG AAG GAA GGT GCG CCT CTG CCT TTT TCT TAC GAT ATC TTG TCA AAC GCG

TTC CAG TAC GGA AAC AGA GCA TTG ACA AAA TAC CCA GAC GAT ATA GCA GAC

TAT TTC AAG CAG TCG TTT CCC GAG GGA TAT TCC TGG GAA AGA ACC ATG ACT

TTT GAA GAC AAA GGC ATT GTC AAA GTG AAA AGT GAC ATA AGC ATG GAG GAA

GAC TCC TTT ATC TAT GAA ATT CGT TTT GAT GGG ATG AAC TTT CCT CCC AAT

GGT CCG GTT ATG CAG AAA AAA ACT TTG AAG TGG GAA CCA TCC ACT GAG ATT

ATG TAC GTG CGT GAT GGA GTG CTG GTC GGA GAT ATT AGC CAT TCT CTG TTG

CTG GAG GGA GGT GGC CAT TAC CGA TGT GAC TTC AAA AGT ATT TAC AAA GCA

AAA AAA GTT GTC AAA TTG CCA GAC TAT CAC TTT GTG GAC CAT CGC ATT GAG

ATC TTG AAC CAT GAC AAG GAT TAC AAC AAA GTA ACG CTG TAT GAG AAT GCA

GTT GCT CGC TAT TCT TTG CTG CCA AGT CAG GCC TAG
```

(2) INFORMATION FOR SEQ. ID. NO.7:

(i) SEQUENCE CHARACTERISTICS:

(A) LENGTH: 225 amino acids
(B) TYPE: amino acid
(C) STRANDEDNESS: single
(D) TOPOLOGY: linear

(ii) MOLECULE TYPE: protein
(ix) FEATURE:

(A) NAME / KEY: Coding Sequence
(B) LOCATION: 1......225

(xi) SEQUENCE DESCRIPTION: SEQ. ID. NO.7:

```
Met Arg Ser Ser Lys Asn Val Ile Lys Glu Phe Met Arg Phe Lys Val Arg
Met Glu

Gly Thr Val Asn Gly His Glu Phe Glu Ile Glu Gly Glu Gly Glu Gly Arg
Pro Tyr

Glu Gly His Asn Thr Val Lys Leu Lys Val Thr Lys Gly Gly Pro Leu Pro
Phe Ala

Trp Asp Ile Leu Ser Pro Gln Phe Gln Tyr Gly Ser Lys Val Tyr Val Lys
His Pro

Ala Asp Ile Pro Asp Tyr Lys Lys Leu Ser Phe Pro Glu Gly Phe Lys Trp
Glu Arg

Val Met Asn Phe Glu Asp Gly Gly Val Val Thr Val Thr Gln Asp Ser Ser
Leu Gln

Asp Gly Cys Phe Ile Tyr Lys Val Lys Phe Ile Gly Val Asn Phe Pro Ser
Asp Gly

Pro Val Met Gln Lys Lys Thr Met Gly Trp Glu Ala Ser Thr Glu Arg Leu
Tyr Pro

Arg Asp Gly Val Leu Lys Gly Glu Ile His Lys Ala Leu Lys Leu Lys Asp
Gly Gly

His Tyr Leu Val Glu Phe Lys Ser Ile Tyr Met Ala Lys Lys Pro Val Gln
Leu Pro

Gly Tyr Tyr Tyr Val Asp Ser Lys Leu Asp Ile Thr Ser His Asn Glu Asp
Tyr Thr

Ile Val Glu Gln Tyr Glu Arg Thr Glu Gly Arg His His Leu Phe Leu
```

(2) INFORMATION FOR SEQ. ID. NO.8:

    (i) SEQUENCE CHARACTERISTICS:

        (A) LENGTH: 681 base pairs
        (B) TYPE: nucleic acid
        (C) STRANDEDNESS: double
        (D) TOPOLOGY: linear

    (ii) MOLECULE TYPE: synthetic
    (ix) FEATURE:

        (A) NAME / KEY: Coding Sequence
        (B) LOCATION: 1.....681

    (xi) SEQUENCE DESCRIPTION: SEQ. ID. NO.8:

```
ATG GTG AGG AGC AGC AAG AAC GTG ATC AAG GAG TTC ATG AGG TTC AAG GTG

CGC ATG GAG GGC ACC GTG AAC GGC CAC GAG TTC GAG ATC GAG GGC GAG GGC

GAG GGC AGG CCC TAC GAG GGC CAC AAC ACC GTG AAG CTT AAG GTG ACC AAG

GGC GGC CCC CTG CCC TTC GCC TGG GAC ATC CTG AGC CCC CAG TTC CAG TAC

GGC AGC AAG GTG TAC GTG AAG CAC CCC GCC GAC ATC CCC GAC TAC AAG AAG

CTG AGC TTC CCC GAG GGC TTC AAG TGG GAG AGG GTG ATG AAC TTC GAG GAC

GGC GGC GTG GTG ACC GTG ACC CAG GAC AGC AGC CTG CAG GAC GGC TGC TTC

ATC TAC AAG GTG AAG TTC ATC GGC GTG AAC TTC CCC AGC GAC GGC CCC GTG

ATG CAG AAG AAG ACC ATG GGC TGG GAG GCC TCC ACC GAG CGC CTG TAC CCC

CGC GAC GGC GTG CTG AAG GGC GAG ATC CAC AAG GCC CTG AAG CTG AAG GAC

GGC GGC CAC TAC CTG GTG GAG TTC AAG TCC ATC TAC ATG GCC AAG AAG CCC

GTG CAG CTG CCC GGC TAC TAC TAC GTG GAC TCC AAG CTG GAC ATC ACC AGC

CAC AAC GAG GAC TAC ACC ATC GTG GAG CAG TAC GAG AGG ACC GAG GGC AGG

CAC CAC CTG TTC CTG TGA
```

(2) INFORMATION FOR SEQ. ID. NO.9:

    (i) SEQUENCE CHARACTERISTICS:

        (A) LENGTH: 226 amino acids
        (B) TYPE: amino acid
        (C) STRANDEDNESS: single
        (D) TOPOLOGY: linear

    (ii) MOLECULE TYPE: protein
    (ix) FEATURE:

        (A) NAME / KEY: Coding Sequence
        (B) LOCATION: 1......226

    (xi) SEQUENCE DESCRIPTION: SEQ. ID. NO.9:

Met Val Arg Ser Ser Lys Asn Val Ile Lys Glu Phe Met Arg Phe Lys Val Arg Met

Glu Gly Thr Val Asn Gly His Glu Phe Glu Ile Glu Gly Glu Gly Glu Gly Arg Pro

Tyr Glu Gly His Asn Thr Val Lys Leu Lys Val Thr Lys Gly Gly Pro Leu Pro Phe

Ala Trp Asp Ile Leu Ser Pro Gln Phe Gln Tyr Gly Ser Lys Val Tyr Val Lys His

Pro Ala Asp Ile Pro Asp Tyr Lys Lys Leu Ser Phe Pro Glu Gly Phe Lys Trp Glu

Arg Val Met Asn Phe Glu Asp Gly Gly Val Val Thr Val Thr Gln Asp Ser Ser Leu

Gln Asp Gly Cys Phe Ile Tyr Lys Val Lys Phe Ile Gly Val Asn Phe Pro Ser Asp

Gly Pro Val Met Gln Lys Lys Thr Met Gly Trp Glu Ala Ser Thr Glu Arg Leu Tyr

Pro Arg Asp Gly Val Leu Lys Gly Glu Ile His Lys Ala Leu Lys Leu Lys Asp Gly

Gly His Tyr Leu Val Glu Phe Lys Ser Ile Tyr Met Ala Lys Lys Pro Val Gln Leu

Pro Gly Tyr Tyr Tyr Val Asp Ser Lys Leu Asp Ile Thr Ser His Asn Glu Asp Tyr

Thr Ile Val Glu Gln Tyr Glu Arg Thr Glu Gly Arg His His Leu Phe Leu

(2) INFORMATION FOR SEQ. ID. NO. 10

    (i) SEQUENCE CHARACTERISTICS:

        (A) LENGTH: 720 base pairs
        (B) TYPE: nucleic acid
        (C) STRANDEDNESS: double
        (D) TOPOLOGY: linear

    (ii) MOLECULE TYPE: cDNA
    (ix) FEATURE:

        (A) NAME / KEY: Coding Sequence
        (B) LOCATION: 1....720

    (xi) SEQUENCE DESCRIPTION: SEQ. ID. NO. 10:

```
ATG GTG AGC AAG GGC GAG GAG CTG TTC ACC GGG GTG GTG CCC ATC CTG GTC

GAG CTG GAC GGC GAC GTA AAC GGC CAC AAG TTC AGC GTG TCC GGC GAG GGC

GAG GGC GAT GCC ACC TAC GGC AAG CTG ACC CTG AAG TTC ATC TGC ACC ACC

GGC AAG CTG CCC GTG CCC TGG CCC ACC CTC GTG ACC ACC TTC TCC TAC GGC

GTG CAG TGC TTC AGC CGC TAC CCC GAC CAC ATG AAG CAG CAC GAC TTC TTC

AAG TCC GCC ATG CCC GAA GGC TAC GTC CAG GAG CGC ACC ATC TTC TTC AAG

GAC GAC GGC AAC TAC AAG ACC CGC GCC GAG GTG AAG TTC GAG GGC GAC ACC

CTG GTG AAC CGC ATC GAG CTG AAG GGC ATC GAC TTC AAG GAG GAC GGC AAC

ATC CTG GGG CAC AAC CTG GAG TAC AAC TAC AAC AGC CAC AAC GTC TAT ATC

ATG GCC GAC AAG CAG AAG AAC GGC ATC AAG GTG AAC TTC AAG ATC CGC CAC

AAC ATC GAG GAC GGC AGC GTG CAG CTC GCC GAC CAC TAC CAG CAG AAC ACC

CCC ATC GGC GAC GGC CCC GTG CTG CTG CCC GAC AAC CAC TAC CTG AGC ACC

CAG TCC GCC CTG AGC AAA GAC CCC AAC GAG AAG CGC GAT CAC ATG GTC CTG

CTG GAG TTC GTG ACC GCC GCC GGG ATC ACT CTC GGC ATG GAC GAG CTG TAC

AAG TAA
```

**Description**

**FIELD OF THE INVENTION**

The present invention relates generally to functional mutants of red fluorescent proteins, and methods for their use.

**BACKGROUND OF THE INVENTION**

Naturally fluorescent proteins are attractive as reporter molecules for cell based assays because of their bright visible fluorescence and ability to be expressed within living cells without the need to add exogenous co-factors or reagents. Fluorescent proteins have been successfully exploited as markers of gene expression, tracers of cell lineage, fusion tags to monitor protein localization within living cells, and as fluorescent donors or acceptors for assays based on the use of fluorescent resonance energy transfer (FRET). Naturally fluorescent proteins have been characterized from a large number of species, however the green fluorescent protein from *Aequorea victoria* is probably the most extensively studied example.

*Aequorea* green fluorescent protein (GFP) is a stable, proteolysis-resistant single polypeptide chain of 238 residues, and has two absorption maxima at around 395 and 475 nm (Tsien (1998) Annu. Rev. Biochem. 67 509-544). The relative amplitudes of these two peaks are sensitive to environmental factors (Ward & Bokman (1982) Biochemistry 21:4535-4540, Ward et al. (1982) Photochem. Photobiol. 35 803-808) and illumination history (A. B. Cubitt et al. (1995) Trends Biochem. Sci. 20 448-455). Excitation at the primary absorption peak of 395 nm yields an emission maximum at 508 nm with a quantum yield of 0.72-0.85 (Shimomura and Johnson (1962) J. Cell. Comp. Physio. 59 223).

The fluorophore results from the autocatalytic cyclization of the polypeptide backbone between residues $Ser^{65}$ and $Gly^{67}$ and oxidation of the $\alpha$-$\beta$ bond of $Tyr^{66}$ (Cody et al., (1993) Biochemistry 32 1212-1218, Heim et al.,(1994) Proc. Natl. Acad. Sci. USA 91 12501-12504). Mutation of $Ser^{65}$ to Thr (S65T) simplifies the excitation spectrum to a single peak at 488 nm of enhanced amplitude (Heim et al., (1995) Nature 373 664-665), which no longer gives signs of conformational isomers. The cDNA for the protein was cloned in 1992 and the protein has been extensively mutated (D.C. Prasher et al., (1992) Gene 111 229-33). Mutagenesis of GFP has resulted in the creation of a variety of mutants that have distinct spectral properties, improved brightness and enhanced expression and folding in mammalian cells compared to the native GFP, (SEQ. ID. NO.: 10), **Table 1.** (Green Fluorescent Proteins, Chapter 2, pages 19 to 47, edited Sullivan and Kay, Academic Press, U.S. Patent Nos: 5,625,048 to Tsien et al., issued April 29, 1997; 5,777,079 to Tsien et al., issued July 7, 1998; and U.S. Patent No. 5,804,387 to Cormack et al., issued September 8, 1998). In many cases, these functional engineered fluorescent proteins have superior spectral properties to wild-type *Aequorea* GFP, and are preferred for use herein.

| **TABLE 1** Mutants of Aequorea Green Fluorescent Proteins | | | | | |
|---|---|---|---|---|---|
| Mutations | Common Name | Quantum Yield ($\Phi$) & Molar Extinction ($\varepsilon$) | Excitation & Emission Max | Relative Fluorescence At 37 °C | Sensitivity To Low pH % max F at pH 6 |
| **S65T type** | | | | | |
| S65T, S72A, N149K, M153T, I167T | Emerald (SEQ. ID. NO.: 28) | $\Phi$ = 0.68 $\varepsilon$ = 57,500 | 487 509 | 100 | 91 |
| F64L, S65T, V163A | | $\Phi$ = 0.58 e = 42,000 | 488 511 | 54 | 43 |
| F64L,S65T | EGFP | $\Phi$ = 0.60 $\varepsilon$ = 55,900 | 488 507 | 20 | 57 |
| S65T | | $\Phi$ = 0.64 $\varepsilon$ = 52,000 | 489 511 | 12 | 56 |
| **Y66H type** | | | | | |
| F64L, Y66H, | P4-3E | $\Phi$ = 0.27 | 384 | 100 | N.D. |

(continued)

| Y66H type | | | | | |
| --- | --- | --- | --- | --- | --- |
| Y145F, V163A | | ε = 22,000 | 448 | | |
| F64L, Y66H, Y145F | | Φ = 0.26  ε = 26,300 | 383  447 | 82 | 57 |
| Y66H, Y145F | P4-3 | Φ = 0.3  ε = 22,300 | 382  446 | 51 | |
| Y66H | BFP | Φ = 0.24  ε = 21,000 | 384  448 | 15 | 59 |
| **Y66W type** | | | | | |
| S65A, Y66W, S72A, N146I, M153T, V163A | W1C | Φ = 0.39  ε = 21,200 | 435  495 | 100 | 82 |
| F64L,S65T, Y66W, N146I, M153T, V163A | W1B | Φ = 0.4  ε = 32,500 | 434 452  476 (505) | 80 | 71 |
| Y66W, N146I, M153T, V163A | hW7 | Φ = 0.42  ε = 23,900 | 434 452  476 (505) | 61 | 88 |
| Y66W | | | 436  485 | N.D. | N.D. |
| **T203Y type** | | | | | |
| S65G, S72A, K79R, T203Y | Topaz | Φ = 0.60  ε = 94,500 | 514  527 | 100 | 14 |
| S65G, V68L, S72A, T203Y | 10C | Φ = 0.61  ε = 83,400 | 514  527 | 58 | 21 |
| S65G,V68L, Q69K, S72A, T203Y | h10C+ | Φ = 0.71  ε = 62,000 | 516  529 | 50 | 54 |
| S65G, S72A, T203H | | Φ = 0.78  ε = 48,500 | 508  518 | 12 | 30 |
| S65G, S72A T203F | | Φ = 0.70  ε = 65,500 | 512  522 | 6 | 28 |
| **T203I type** | | | | | |
| T203I, S72A, Y145F | Sapphire | Φ= 0.64  ε = 29,000 | 395  511 | 100 | 90 |
| T203I T202F | H9 | Φ = 0.6  ε = 20,000 | 395  511 | 13 | 80 |

X-ray crystallographic studies have clarified the protein structure and helped to elucidate the effect of mutations, environmental effects, and photochemical events that occur in wild-type and mutant forms of *Aequorea* GFP (Ormo et al., (1996) Science 273 1392-1395, Yang et al., (1996) Nat. Biotechnol. 14 1246-1251, Brejc et al., (1997) Proc. Natl. Acad. Sci. USA 94 2306-2311, Scharnagl et al., (1999) Biophys J. 77 1839-1857, Elsliger et al. (1999) Biochem. 38 5296-5301). These studies have provided a detailed molecular picture of the chromophore structure in *Aequorea* GFP and have enabled a precise understanding of how changes in the electronic environment around the chromophore lead to altered fluorescent properties.

Despite this unique understanding, current efforts to date have failed to create stable, well-defined, red fluorescent mutants of *Aequorea* GFP. Red fluorescent proteins (RFPs) are particularly attractive as fluorescent markers because red light is less phototoxic, is transmitted through tissues more efficiently, and is less scattered than blue or UV light sources. Additionally cells typically exhibit less autofluorescence when illuminated with red light compared to UV light.

Recently Anthozoan fluorescent proteins isolated from a number of species of coral (Matz et al., (1999) Nature Biotech. 17 969-973), and these proteins have been the focus of much attention because they exhibit fluorescent emission spectra at red wavelengths.

However, the existing wild type Anthozoan fluorescent proteins are not well suited for many applications because of their broad excitation and emission spectra, relatively small stokes shift, and poor quantum yield and molar extinction coefficient when expressed in mammalian cells. The broad excitation spectra result in significant spectral overlap of the red fluorescent protein with the spectra of other available fluorescent proteins, and makes it difficult to efficiently excite the red fluorescent protein without also directly exciting other fluorescent proteins. These factors reduce the effectiveness of the existing red fluorescent proteins for multiplexed analysis and FRET applications.

The present invention relates to functional red fluorescent proteins that are designed to have improved brightness, reduced spectral cross talk and to be rapidly and efficiently expressed in mammalian cells. Functional red fluorescent proteins are well suited for multiplexed fluorescent analysis, and FRET based applications with existing *Aequorea* fluorescent proteins.

## SUMMARY OF THE INVENTION

The present invention includes mutants of red fluorescent proteins with improved spectral, and biochemical properties, for use as fluorescent markers and as FRET partners. The functional red fluorescent proteins of the present invention comprise one or more key mutations designed to provide for improved folding, brightness and to create functional red fluorescent proteins that have sharper, more defined excitation and emission peaks when expressed in mammalian cells.

In one embodiment this invention provides a nucleic acid comprising a nucleotide sequence encoding a functional red fluorescent protein comprising at least one mutation, as set forth in claim 1.

In one aspect, the functional red fluorescent protein exhibits a reduced molar extinction coefficient at 487 nm compared to the wild type Anthozoan red fluorescent protein (SEQ. ID. NO. 7).

In one aspect, the functional red fluorescent protein exhibits a reduced molar extinction coefficient at 530 nm compared to the wild type Anthozoan red fluorescent protein (SEQ. ID. NO. 7).

In one aspect, the functional red fluorescent protein exhibits a higher molar extinction coefficient at 583 nm compared to the wild type Anthozoan red fluorescent protein (SEQ. ID. NO. 7).

In one aspect, the functional red fluorescent protein is brighter than the wild type Anthozoan red fluorescent protein (SEQ. ID. NO. 7) when excited at 558 nm.

In one aspect, the functional red fluorescent protein is brighter than the wild type Anthozoan red fluorescent protein (SEQ. ID. NO. 7) when expressed in a mammalian cell grown at 37 °C.

In another aspect, the functional red fluorescent protein exhibits a higher quantum yield compared to the wild type Anthozoan red fluorescent protein (SEQ. ID. NO. 7).

In one aspect, the functional red fluorescent protein exhibits a faster rate of autocatalytic formation compared to the wild type Anthozoan red fluorescent protein (SEQ. ID. NO. 7).

In one embodiment, the functional red fluorescent protein comprises at least one mutation corresponding to position 64 in SEQ. ID. NO. 7 selected from the group consisting of Q64K, Q64P, Q64T, Q64N and Q64R.

In one embodiment, the functional red fluorescent protein comprises at least one mutation corresponding to position 65 in SEQ. ID. NO. 7 selected from the group consisting of F65L, F65V, F65I, F65M, F65Y and F65W.

In one embodiment, the functional red fluorescent protein comprises at least one mutation corresponding to position 66 in SEQ. ID. NO. 7 selected from the group consisting of Q66R, Q66R, Q66P, Q66K, Q66E, Q66T, Q66A and Q66G.

In one embodiment, the functional red fluorescent protein comprises at least one mutation corresponding to position 69 in SEQ. ID. NO. 7 selected from the group consisting of S69L, S69A, S69V and S69T.

In one embodiment, the functional red fluorescent protein comprises at least one mutation corresponding to position 70 in SEQ. ID. NO. 7 selected from the group consisting of K70M, K70Q, K70L and K70R

In one embodiment, the functional red fluorescent protein comprises at least one mutation corresponding to position 71 in SEQ. ID. NO. 7 selected from the group consisting of V71C, V71L, V71A and V71I.

In one embodiment, the functional red fluorescent protein comprises at least one mutation corresponding to position 72 in SEQ. ID. NO. 7 selected from the group consisting of Y72F and Y72W.

In one embodiment, the functional red fluorescent protein comprises at least one mutation corresponding to position 73 in SEQ. ID. NO. 7 selected from the group consisting of V73A, V73L, V73S and V73I.

In one embodiment, the functional red fluorescent protein comprises at least one mutation corresponding to position 145 in SEQ. ID. NO. 7 selected from the group consisting of A145P, A145S, A145G and A145L.

In one embodiment, the functional red fluorescent protein comprises at least one mutation corresponding to position 147 in SEQ. ID. NO. 7 selected from the group consisting of T147N, T147K and T147S.

In one embodiment, the functional red fluorescent protein comprises at least one mutation corresponding to position 163 in SEQ. ID. NO. 7 selected from the group consisting ofK163I, K163R, K163T, K163E, K163V, K163G and K163A.

In one embodiment, the functional red fluorescent protein comprises at least one mutation corresponding to position 179 in SEQ. ID. NO. 7 selected from the group consisting of S 179A, S 179P, S 179T, S 179E, S 179Q and S 179K.

In one embodiment, the functional red fluorescent protein comprises at least one mutation corresponding to position 197 in SEQ. ID. NO. 7 selected from the group consisting of S197Y, S 197T, S197N and S197A.

In one embodiment, the functional red fluorescent protein comprises at least one mutation corresponding to position 216 in SEQ. ID. NO. 7 selected from the group consisting of R216K, R216L, R216C and R216F.

In one embodiment the invention comprises an expression vector, comprising; expression control sequences operatively linked to a nucleic acid molecule encoding a functional red fluorescent protein whose sequence differs from the amino acid sequence of an Anthozoan red fluorescent protein (SEQ. ID. NO. 7) by at least one amino acid substitution set forth in claim 1.

In another embodiment, the invention includes a recombinant host cell, comprising; a nucleic acid molecule encoding a functional red fluorescent protein whose sequence differs from the amino acid sequence of an Anthozoan red fluorescent protein (SEQ. ID. NO. 7) by at least one amino acid substitution set forth in claim 1.

In yet another embodiment, the invention comprises a functional fluorescent protein, comprising; an amino acid sequence that differs from the amino acid sequence of an Anthozoan red fluorescent protein (SEQ. ID. NO. 7) by at least one amino acid substitution set forth in claim 1.

In another aspect the invention includes a fusion protein, comprising; a protein of interest operably coupled to a functional red fluorescent protein whose sequence differs from the amino acid sequence of an Anthozoan red fluorescent protein (SEQ. ID. NO. 7) by at least one amino acid substitution set forth in claim 1.

In one embodiment the invention includes a non-human transgenic organism, comprising; a nucleic acid molecule encoding a functional red fluorescent protein whose sequence differs from the amino acid sequence of an Anthozoan red fluorescent protein (SEQ. ID. NO. 7) by at least one amino acid substitution set forth in claim 1.

In another aspect, the invention includes a method for identifying a protein - protein interaction, comprising;

a) providing a population of cells comprising,
a functional red fluorescent protein whose sequence differs from the amino acid sequence of an Anthozoan red fluorescent protein (SEQ. ID. NO. 7) by at least one amino acid substitution set forth in claim 1, wherein said functional red fluorescent protein is operably coupled to a first protein of interest,
b) introducing a library of test proteins of interest operably coupled to a functional green fluorescent protein into said population of cells,
wherein said functional green fluorescent protein and said functional red fluorescent protein can undergo fluorescence energy transfer (FRET), and
wherein each member of said population of cells receives on average one member of said library of test proteins of interest operably coupled to said functional green fluorescent protein,
c) screening said population of cells for FRET between said functional green fluorescent protein and said functional red fluorescent protein, and
d) comparing the FRET in step c) to the FRET in a control cell in the absence of said library of test proteins of interest operably coupled to said functional green fluorescent protein.

In another embodiment, the invention includes a method for identifying a modulator of protein - protein interactions, comprising;

a) contacting a cell with a test chemical, wherein said cell comprises,

i) a functional red fluorescent protein whose sequence differs from the amino acid sequence of an Anthozoan red fluorescent protein (SEQ. ID. NO. 7) by at least one amino acid substitution set forth in claim 1, wherein said functional red fluorescent protein is operably coupled to a first protein of interest,
ii) a functional green fluorescent protein, wherein said functional green fluorescent protein is operably coupled to a second protein of interest, and wherein said functional green fluorescent protein and said functional red fluorescent protein undergo fluorescence energy transfer (FRET) when said first operably coupled protein of interest and said second operably protein of interest associate,

b) detecting FRET between said functional green fluorescent protein and said functional red fluorescent protein in the presence of said test chemical, and

c) comparing the FRET in step b) to the FRET in a control cell in the absence of said test chemical.

In one aspect of this method, the method further comprises the step of contacting the cell with an activator prior to the addition of the test chemical. In another aspect the method further includes the step of detecting the viability of the cell.

## BRIEF DESCRIPTION OF THE FIGURES

FIG. 1 Shows the mammalianized RFP created to provide for optimal codon usage and translational initiation in mammalian cells. Restriction sites, for insertion of mutagenic oligonucleotides, are shown above the sequence.

FIG. 2. Shows the retroviral mammalian expression vector ABSC258. In this construct high-level mammalian expression is achieved via the strong viral CMV promoter.

FIG. 3. Shows the result of flow cytometry analysis of wild type and RFP expressing NIH3T6 cells.

## DETAILED DESCRIPTION OF THE PREFERRED EMBODIMENTS

*Definitions*

The techniques and procedures are generally performed according to conventional methods in the art and various general references. (Lakowicz, J.R. Topics in Fluorescence Spectroscopy, (3 volumes) New York: Plenum Press (1991), and Lakowicz, J. R. (1996) Scanning Microsc SuppL 10 213-24, for fluorescence techniques; Sambrook et al. (1989) Molecular Cloning: A Laboratory Manual, 2nd ed. Cold Spring Harbor Laboratory Press, Cold Spring Harbor, N.Y., for molecular biology methods; Cells: A Laboratory Manual, 1st edition (1998) Cold Spring Harbor Laboratory Press, Cold Spring Harbor, N.Y., for cell biology methods; Optics Guide 5 Melles Griot® Irvine CA, and Optical Waveguide Theory, Snyder & Love published by Chapman & Hall for general optical methods.

"Activity" refers to the enzymatic or non-enzymatic activity capable of modifying an amino acid residue or peptide bond (preferably enzymatic). Such covalent modifications include proteolysis, phosphorylation, dephosphorylation, glycosylation, methylation, sulfation, prenylation and ADP-ribsoylation. The term includes non-covalent modifications including protein-protein interactions, and the binding of allosteric, or other modulators or second messengers such as calcium, or cAMP or inositol phosphates to a polypeptide.

Amino acid "substitutions" are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid "insertions" or "deletions" are changes to or within an amino acid sequence. They typically fall in the range of about 1 to 5 amino acids. The variation allowed in a particular amino acid sequence may be experimentally determined by producing the peptide synthetically or by systematically making insertions, deletions, or substitutions of nucleotides in the gene sequence using recombinant DNA techniques.

"Animal" as used herein may be defined to include human, domestic (cats, dogs, etc), agricultural (cows, horses, sheep, goats, chicken, fish, etc) or test species (frogs, mice, rats, rabbits, simians, etc).

"Chimeric" molecules are polynucleotides or polypeptides which are created by combining one or more nucleotide sequences of this invention (or their parts) with additional nucleic acid sequence(s). Such combined sequences may be introduced into an appropriate vector and expressed to give rise to a chimeric polypeptide which may be expected to be different from the native molecule in one or more of the following characteristics: cellular location, distribution, ligand- binding affinities, interchain affinities, degradation/turnover rate, signaling, etc.

The terms "cleavage site" or "protease site" refers to the bond cleaved by the protease (e.g. a scissile bond) and typically the surrounding three to four amino acids of either side of the bond.

"Control elements" or "regulatory sequences" are those non-translated regions of the gene or DNA such as enhancers, promoters, introns and 3' untranslated regions which interact with cellular proteins to carry out replication, transcription, and translation. They may occur as boundary sequences or even split the gene. They function at the molecular level and along with regulatory genes are very important in development, growth, differentiation and aging processes.

"Corresponds to" refers to a polynucleotide sequence that is homologous (i.e., is identical, not strictly evolutionarily related) to all or a portion of a reference polynucleotide sequence, or that a polypeptide sequence is identical to all or a portion of a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

"Derivative" refers to those polypeptides which have been chemically modified by such techniques as ubiquitination, labeling, pegylation (derivatization with polyethylene glycol), and chemical insertion or substitution of amino acids such as ornithine which do not normally occur in human proteins.

The term "engineered protease site" refers to a protease site that has been modified from the naturally existing sequence by at least one amino acid substitution.

The term "fluorescent property" refers to any one of the following, the molar extinction coefficient at an appropriate excitation wavelength, the fluorescent quantum efficiency, the shape of the excitation or emission spectrum, the excitation wavelength maximum, or the emission magnitude at any wavelength during, or at one or more times after excitation of the fluorescent moiety, the ratio of excitation amplitudes at two different wavelengths, the ratio of emission amplitudes at two different wavelengths, the excited state lifetime, the fluorescent anisotropy or any other measurable property of a fluorescent moiety and the like. Preferably fluorescent property refers to fluorescence emission, or the fluorescence emission ratio at two or more wavelengths.

The term "homolog" refers to two sequences or parts thereof, that are greater than, or equal to 85% identical when optimally aligned using the ALIGN program. Homology or sequence identity refers to the following. Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of more than 5 (in standard deviation units) using the program ALIGN with the mutation data matrix and a gap penalty of 6 or greater. See Dayhoff, (1972) in Atlas of Protein Sequence and Structure 5, National Biomedical Research Foundation, 101-110, and Supplement 2 to this volume, pp. 1-10.

An "inhibitor" is a substance that retards or prevents a chemical or physiological reaction or response. Common inhibitors include but are not limited to antisense molecules, antibodies, antagonists and their derivatives.

"Isolated" refers to material removed from its original environment (e.g. the natural environment if it is naturally occurring), and thus is altered from its natural state. For example, an isolated polynucleotide could be part of a vector or a composition of matter, or could be contained within a cell, and still be "isolated" because that vector, composition of matter, or particular cell is not the original environment of the polynucleotide.

The term "linker" or "linker moiety" refers to an amino acid, polypeptide or protein sequence that serves to operatively couple a fluorescent protein to a protein of interest or second fluorescent protein. Linkers typically comprise a single polypeptide chain that covalently couples the fluorescent protein to the protein of interest or second fluorescent protein. Linkers may be of any size.

The term "modulates" refers to, either the partial or complete, enhancement or inhibition (e.g. attenuation of the rate or efficiency) of an activity or process.

The term "modulator" refers to a chemical compound (naturally occurring or non-naturally occurring), such as a biological macromolecule (e.g., nucleic acid, protein, non-peptide, or organic molecule), or an extract made from biological materials such as bacteria, plants, fungi, or animal (particularly mammalian, including human) cells or tissues. Modulators are evaluated for potential activity as inhibitors or activators (directly or indirectly) of a biological process or processes (e.g., agonist, partial antagonist, partial agonist, inverse agonist, antagonist, antineoplastic agents, cytotoxic agents, inhibitors of neoplastic transformation or cell proliferation, cell proliferation-promoting agents, and the like) by inclusion in screening assays described herein. The activity of a modulator may be known, unknown or partially known.

"Naturally fluorescent protein" refers to proteins capable of forming a highly fluorescent, intrinsic chromophore either through the cyclization and oxidation of internal amino acids within the protein or via the enzymatic addition of a fluorescent co-factor. Typically such chromophores can be spectrally resolved from weakly fluorescent amino acids such as tryptophan and tyrosine.

An "oligonucleotide" or "oligomer" is a stretch of nucleotide residues which has a sufficient number of bases to be used in a polymerase chain reaction (PCR), a site directed mutagenesis reaction or a cassette to create a desired sequence element. These short sequences are based on (or designed from) genomic or cDNA sequences and are used to amplify, mutate or create particular sequence elements. Oligonucleotides or oligomers comprise portions of a DNA sequence having at least about 10 nucleotides and as many as about 50 nucleotides, preferably about 15 to 30 nucleotides. They are chemically synthesized and may also be used as probes.

An "oligopeptide" is a short stretch of amino acid residues and may be expressed from an oligonucleotide. It may be functionally equivalent to and either the same length as or considerably shorter than a "fragment ", "portion ", or "segment" of a polypeptide. Such sequences comprise a stretch of amino acid residues of at least about 5 amino acids and often about 17 or more amino acids, typically at least about 9 to 13 amino acids, and of sufficient length to display biologic and/or immunogenic activity.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences.

The term "operably coupled" refers to a juxtaposition wherein the components so described are either directly or indirectly coupled. Examples of directly coupled components include proteins that are translationally fused together. Examples of indirectly coupled components include proteins that can functionally associate either transiently, or persistently, through a binding interaction.

The term "polynucleotide" refers to a polymeric form of nucleotides of at least 10 bases in length, either ribonucleotides or deoxynucleotides. Modified forms and analogs of either type of nucleotide are also included, as are ribonucleotides or deoxynucleotides linked via novel bonds such as those described in U.S. Patent No. 5,532,130, European Patent Applications EP 0 839 830, EP 0 742 287, EP 0 285 057 and EP 0 694 559. The term includes single and double stranded forms of nucleotides, or a mixture of single and double stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine, as well as other chemical or enzymatic modifications.

The term "polypeptide" refers to amino acids joined to each other by peptide bonds or modified peptide bonds, i.e. peptide isosteres, and may contain amino acids other than the 20 gene-encoded amino acids. The polypeptides may be modified by either natural processes, such as posttranslational processing, or by chemical modification techniques which are well known in the art. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Modification include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of a phosphatidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristolyation, oxidation, pergylation, proteolytic processing, phosphorylation, prenylation, racemization, selenoylation, sulfation, transfer-RNA mediated addition of amino acids to protein such as arginylation. (See Proteins- Structure and Molecular Properties 2nd Ed., T.E. Creighton, W.H. Freeman and Company, New York (1993); Posttranslational Covalent Modification of Proteins, B.C. Johnson, Ed., Academic Pres, New York, pp. 1-12 (1983).

A "portion" or "fragment" of a polynucleotide or nucleic acid comprises all or any part of the nucleotide sequence having fewer nucleotides than about 6 kb, preferably fewer than about 1 kb which can be used as a probe. Such probes may be labeled with reporter molecules using nick translation, Klenow fill-in reaction, PCR or other methods well known in the art. After pretesting to optimize reaction conditions and to eliminate false positives, nucleic acid probes may be used in Southern, northern or in situ hybridizations to determine whether DNA or RNA encoding the protein is present in a biological sample, cell type, tissue, organ or organism.

"Probes" are nucleic acid sequences of variable length, preferably between at least about 10 and as many as about 6,000 nucleotides, depending on use. They are used in the detection of identical, similar, or complementary nucleic acid sequences. Longer length probes are usually obtained from a natural or recombinant source, are highly specific and much slower to hybridize than oligomers. They may be single- or double-stranded and carefully designed to have specificity in PCR, hybridization membrane-based, or ELISA-like technologies.

The term "recognition motif" refers to all or part of a polypeptide sequence recognized by a post-translational modification activity to enable a polypeptide to become modified by that post-translational modification activity. Typically, the affinity of a protein, e.g. enzyme, for the recognition motif is about 1 mM (apparent $K_d$), preferably a greater affinity of about 10 $\mu$M, more preferably, 1 $\mu$M or most preferably has an apparent $K_d$ of about 0.1 $\mu$M. The term is not meant to be limited to optimal or preferred recognition motifs, but encompasses all sequences that can specifically confer substrate recognition to a peptide. In some embodiments the recognition motif is a phosphorylated recognition motif (e.g. includes a phosphate group), or comprises other post-translationally modified residues.

"Recombinant nucleotide variants" are polynucleotides that encode a protein. They may be synthesized by making use of the "redundancy" in the genetic code. Various codon substitutions, such as the silent changes which produce specific restriction sites or codon usage-specific mutations, may be introduced to optimize cloning into a plasmid or viral vector or expression in a particular prokaryotic or eukaryotic host system, respectively.

"Recombinant polypeptide variant" refers to any polypeptide which differs from a naturally occurring polypeptide by amino acid insertions, deletions and/or substitutions, created using recombinant DNA techniques. Guidance in determining which amino acid residues may be replaced, added or deleted without abolishing characteristics of interest may be found by comparing the sequence of a polypeptide with that of related polypeptides and minimizing the number of amino acid sequence changes made in highly conserved regions.

A "signal or leader sequence" is a short amino acid sequence which is or can be used, when desired, to direct the polypeptide through a membrane of a cell. Such a sequence may be naturally present on the polypeptides of the present invention or provided from heterologous sources by recombinant DNA techniques.

A "standard" is a quantitative or qualitative measurement for comparison. Preferably, it is based on a statistically appropriate number of samples and is created to use as a basis of comparison when performing diagnostic assays, running clinical trials, or following patient treatment profiles. The samples of a particular standard may be normal or similarly abnormal.

The term "stringent hybridization conditions", refers to an overnight incubation at 42 °C in a solution comprising 50 % formamide, 5x SSC (750 mM NaCl, 75 mM sodium citrate), 50 mM sodium phosphate (pH 7.6), 5x Denhardt's solution, 10 % dextran sulfate and 20 $\mu$g/ml denatured sheared salmon sperm DNA, followed by washing the filters in O.lx SSC at about 65 °C. Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lower stringency); salt conditions, or temperature. For example, lower stringency conditions include an overnight incubation at 37 °C in a solution comprising 6x SSPE (20X SSPE=3M NaCl; 0.2M NaH2PO4; 0.02M EDTA, pH 7.4), 0.5% SDS, 30 % formamide, 100 $\mu$g/ml salmon sperm blocking DNA; followed by washes at 50 °C with 1XSSPE, 0.1% SDS. In addition, to achieve even lower stringency, washes performed following stringent hybridization can be done at higher salt concentrations (e.g. 5X SSC). Variation in the above conditions may be accomplished through the inclusion and / or substitution of alternative blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. A polynucleotide which hybridizes only to polyA+ sequences (such as any 3' terminal polyA+ tract of a cDNA shown in the sequence listing), or to a complementary stretch of T (or U) residues would not be included in the definition of a "polynucleotide" since such a polynucleotide would hybridize to any nucleic acid molecule containing a poly (A) stretch, or the complement thereof.

The term "target" refers to a biochemical entity involved in a biological process. Targets are typically proteins that play a useful role in the physiology or biology of an organism. A therapeutic chemical binds to a target to alter or modulate its function. As used herein, targets can include cell surface receptors, G-proteins, kinases, ion channels, phopholipases, proteases and other proteins mentioned herein.

The term "test chemical" refers to a chemical to be tested by one or more screening method(s) of the invention as a putative modulator. A test chemical can be any chemical, such as an inorganic chemical, an organic chemical, a protein, a peptide, a carbohydrate, a lipid, or a combination thereof. Usually, various predetermined concentrations of test chemicals are used for screening, such as 0.01 micromolar, 1 micromolar and 10 micromolar. Test chemical controls can include the measurement of a signal in the absence of the test compound or comparison to a compound known to modulate the target.

The term "transgenic" is used to describe an organism that includes exogenous genetic material within all of its cells. The term includes any organism whose genome has been altered by *in vitro* manipulation of the early embryo or fertilized egg or by any transgenic technology to induce a specific gene knockout.

The term "transgene" refers any piece of DNA which is inserted by artifice into a cell, and becomes part of the genome of the organism (i.e., either stably integrated or as a stable extrachromosomal element) which develops from that cell. Such a transgene may include a gene which is partly or entirely heterologous (*i.e.*, foreign) to the transgenic organism, or may represent a gene homologous to an endogenous gene of the organism. Included within this definition is a transgene created by the providing of an RNA sequence that is transcribed into DNA and then incorporated into the genome. The transgenes of the invention include DNA sequences that encode the functional red fluorescent proteins that may be expressed in a transgenic non-human animal.

The following terms are used to describe the sequence relationships between two or more polynucleotides: "reference sequence", "comparison window", "sequence identity", "percentage identical to a sequence", and "substantial identity". A "reference sequence" is a defined sequence used as a basis for a sequence comparison; a reference sequence may be a subset of a larger sequence, for example, as a segment of a full-length cDNA or may comprise a complete cDNA or gene sequence. Generally, a reference sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman (1981) Adv. Appl. Math. 2: 482, by the homology alignment algorithm of Needleman and Wunsch (1970) J. Mol. Biol. 48: 443, by the search for similarity method of Pearson and Lipman (1988) Proc. Natl. Acad. Sci. (U.S.A.) 85: 2444, by computerized implementations of these algorithms (GAP, BESTFIT, FASTA, and TFASTA in the Wisconsin Genetics Software Package Release 7.0, Genetics Computer Group, 575 Science Dr., Madison, WI), or by inspection, and the best alignment (i.e., resulting in the highest percentage of homology over the comparison window) generated by the various methods selected. The term "sequence identity" means that two polynucleotide sequences are identical (i.e., on a nucleotide-by-nucleotide basis) over the window of comparison. The term "percentage identical to a sequence" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 30 percent sequence identity, preferably at least 50 to 60 percent sequence identity, more usually at least 60 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison.

As applied to polypeptides, the term "substantial identity" means that two peptide sequences, when optimally aligned, such as by the programs GAP or BESTFIT using default gap weights, share at least 30 percent sequence identity, preferably at least 40 percent sequence identity, more preferably at least 50 percent sequence identity, and most preferably at least 60 percent sequence identity. Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, glutamic-aspartic, and asparagine-glutamine.

Since the list of technical and scientific terms cannot be all encompassing, any undefined terms shall be construed to have the same meaning as is commonly understood by one of skill in the art to which this invention belongs. Furthermore, the singular forms "a", "an" and "the" include plural referents unless the context clearly dictates otherwise. For example, reference to a "restriction enzyme" or a "high fidelity enzyme" may include mixtures of such enzymes and any other enzymes fitting the stated criteria, or reference to the method includes reference to one or more methods for obtaining cDNA sequences which will be known to those skilled in the art or will become known to them upon reading this specification.

Before the present sequences, variants, formulations and methods for making and using the invention are described, it is to be understood that the invention is not to be limited only to the particular sequences, variants, formulations or methods described. The sequences, variants, formulations and methodologies may vary, and the terminology used herein is for the purpose of describing particular embodiments. The terminology and definitions are not intended to be limiting since the scope of protection will ultimately depend upon the claims.

I. RED FLUORESCENT PROTEINS

Anthozoan fluorescent proteins (SEQ. ID. NOs 1 to 7) isolated from various species of coral display a range of fluorescence properties (**Table 2**) ranging from green fluorescent to red fluorescent emission. Compared to *Aequorea victoria* GFP, the Anthozoan fluorescent proteins exhibit overall sequence identities of between 26 to 30 % identity.

| TABLE 2 Anthozoa Fluorescent Proteins | | | | | |
|---|---|---|---|---|---|
| Species | Protein Name | Quantum Yield ($\Phi$) & Molar Extinction ($\varepsilon$) | Excitation & Emission Max | Relative Brightness | SEQ. ID. NO.: |
| *Anemonia majano* | amFP486 | $\Phi$= 0.24 $\varepsilon$ = 40,000 | 458 486 | 0.43 | SEQ. ID. NO.: 1 |
| *Zoanthus sp* | zFP506 zFP538 | $\Phi$ = 0.63 $\varepsilon$ = 35,600 $\Phi$ = 0.42 $\varepsilon$ = 20,200 | 496, 506 528,538 | 1.02 0.38 | SEQ. ID. NO.:2 SEQ. ID. NO.:3 |
| *Discosoma striata* | dsFP483 | $\Phi$ =0.46 $\varepsilon$ = 23,900 | 443 483 | 0.5 | SEQ. ID. NO.:4 |
| *Discosoma sp "red"* | drFP583 | $\Phi$ =0.23 $\varepsilon$ = 22,500 | 558 583 | 0.24 | SEQ. ID. NO.:5 |
| *Clavularia sp* | CFP484 | $\Phi$ =0.48 $\varepsilon$ = 35,300 | 456 484 | 0.77 | SEQ. ID. NO.:6 |

In spite of the relatively low sequence identity, the alignment of the Anthozoan and *Aequorea* fluorescent proteins is consistent with the possibility that both types of protein share a common overall structural orientation and protein fold. A comparison of the sequences reveals a tendency for amino acids to alternate between hydrophobic and hydrophilic residues along β-strands, and for the conservation of buried hydrophobic core residues, as well as turn motifs.

Compared to *Aequorea* GFP, the red Anthozoan fluorescent proteins have relatively low quantum yields and molar extinction coefficients resulting in proteins that exhibit an overall brightness of approximately one quarter of that of wild type *Aequorea* GFP. The broad excitation and emission spectra of the wild type red fluorescent proteins makes it difficult to selectively excite or observe the proteins for multiplexed analysis or FRET applications.

II. DESIGN OF FUNCTIONAL RED FLUORESCENT PROTEIN MUTANTS

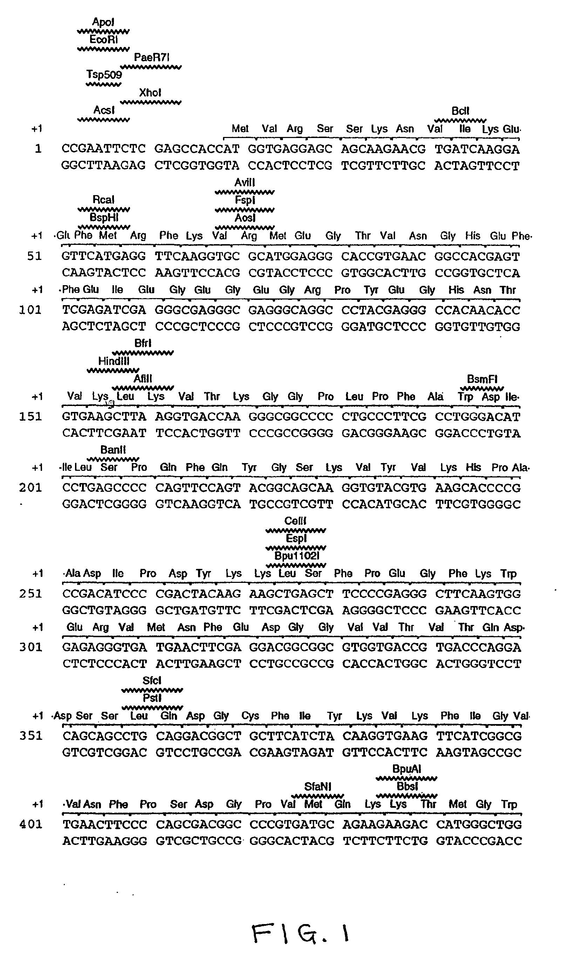

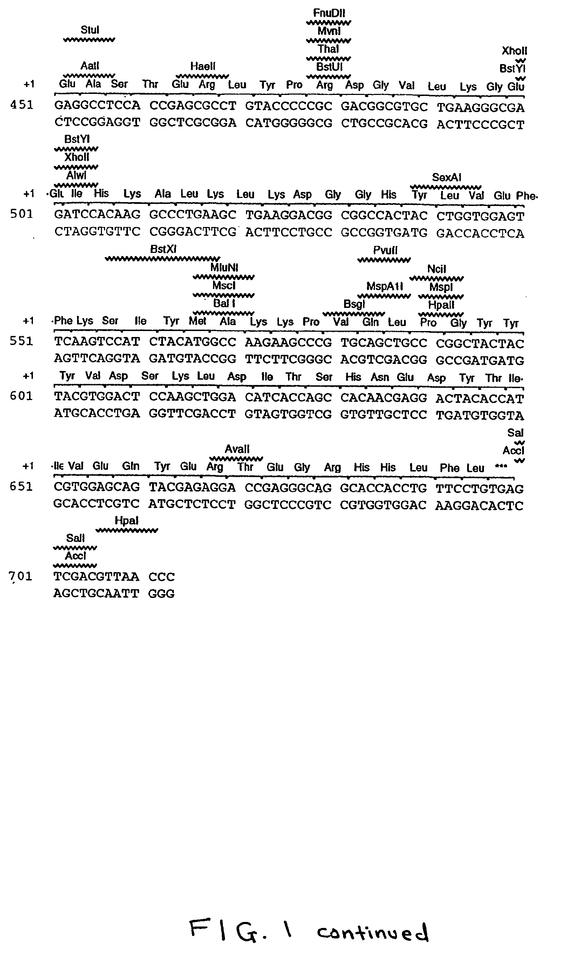

To design improved mutants of the Anthozoan red fluorescent proteins a synthetic protein (SEQ. ID. NO. 8 (nucleotide sequence), & SEQ. ID. NO. 9 (amino acid sequence) was constructed which provided for the ability to clone in a series of oligonucleotides containing randomized nucleic acid sequences at key positions in the red fluorescent protein **(FIG.1).**

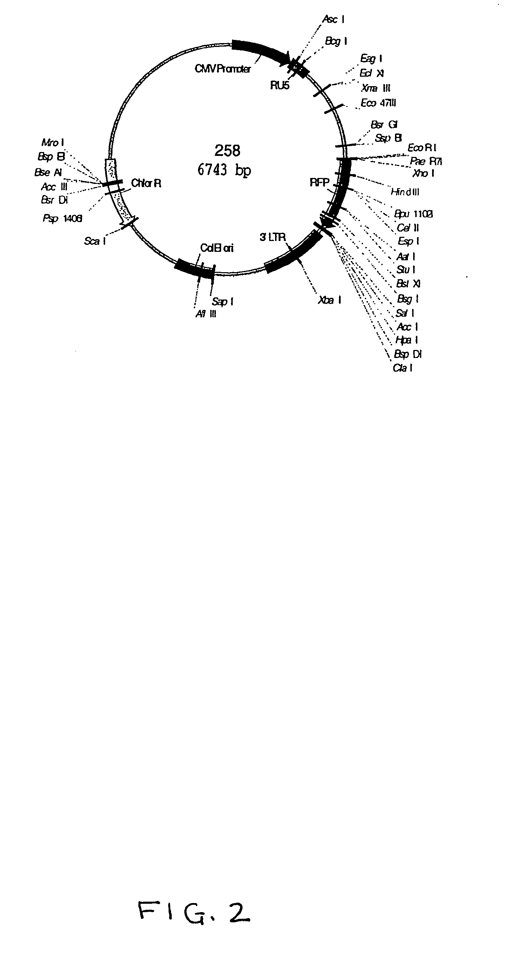

In order to produce functional red fluorescent proteins capable of high level expression in mammalian cells, a synthetic gene encoding the coding region was produced. This sequence contained an additional amino acid (valine) after the start methionine to provide for an optimal Kozak sequence and high level translational initiation. The synthetic red fluorescent protein (SEQ. ID. NO. 9) was constructed by systematically replacing the wild-type codons with codons most frequently used in highly expressed human genes (see U.S. Patent No. 5,795,737, issued August 18, 1998). This synthetic gene was assembled from chemically synthesized oligonucleotides of 70 to 100 bases in length using standard molecular biology methodology. Single stranded oligonucleotide pools were PCR amplified before cloning, and the PCR products purified in agarose gels and used as templates in the next PCR step. Two adjacent fragments were then co-amplified via the use of overlapping sequences at the end of either fragment to build larger fragments. These fragments which were between 350 and 400 bp in size, were sequentially subcloned to assemble the entire gene, **FIG 1.** (Synthetic Genetics, San Diego California). The synthetic gene (SEQ. ID. NO. 9) was then sequenced, and subcloned into the retroviral expression vector ABSC258 (**FIG. 2**).

Retroviral expression vectors provide for highly efficient gene transfer to mammalian cells and stable long-term expression. These characteristics are important to ensure that libraries of mutant red fluorescent proteins can be efficiently introduced into mammalian cells and subsequently analyzed and sequenced.

Mutagenesis of the synthetic gene was completed by sub-cloning mutagenic double stranded oligonucleotide sequences into the synthetic gene (SEQ. ID. NO. 9). These oligonucleotides enabled defined regions of the protein to be targeted for mutagenesis enabling the conservation of the overall structural framework of the protein to remain intact. These oligonucleotides (**Table 3**) were designed to be cassetted into the engineered restriction sites incorporated during synthesis of the synthetic gene.

| TABLE 3 | | | | | | | | | | | | |
|---|---|---|---|---|---|---|---|---|---|---|---|---|
| Relative position in GFP | drFP583 | dsFP483, GFP | Degenerate codon bp Upper case indicates 90% probability, lower case indicates 10 % probability | | | 1st row = Amino acids generated from the selected degenerate codon 2nd row = Codon used 3rd row = Probability | | | | | | |
| 63 | Q64 | T | C | A | G | Q | K | P | T | | | |
| | | | a | c | | CAG | AaG | CgG | acG | | | |
| | | | | | | 0.81 | 0.09 | 0.09 | 0.01 | | | |
| 64 | F65 | L | T | T | C | F | V | I | L | | | |
| | | | a, g | | g | TTC | gTg, gTC | Atc | TTg | | | |
| | | | | | | 0.72 | 0.1 | 0.09 | 0.08 | | | |
| 65 | Q66 | S | C | A | G | Q | R | P | K | E | T | A | G |
| | | | a, g | c, g | | CAG | CgC, agG | CcG | Aag | Gag | acG | gcG | ggG |
| | | | | | | 0.64 | 0.09 | 0.08 | 0.08 | 0.08 | 0.01 | 0.01 | 0.01 |
| 68 | S69 | N,V, L | T | C | G | S | L | A | V | | | |
| | | | g | t | | TCG | TtG | gCG | gtG | | | |
| | | | | | | 0.81 | 0.09 | 0.09 | 0.01 | | | |

| 69 | K70 | Q, M | A | A | G | K | M | Q | L | | | | |
|----|-----|------|---|---|---|---|---|---|---|---|---|---|---|
| | | | c | t | | AAG | AtG | cAG | ctG | | | | |
| | | | | | | 0.81 | 0.09 | 0.09 | 0.01 | | | | |
| 70 | V71 | A, C | G | T | C | V | C | | | | | | |
| | | | | c | | GTC | GcC | | | | | | |
| | | | | | | 0.9 | 0.1 | | | | | | |
| 71 | Y72 | F | T | A | C | Y | F | | | | | | |
| | | | | t | | TAC | TtC | | | | | | |
| | | | | | | 0.9 | 0.1 | | | | | | |
| 72 | V73 | S, A | G | T | G | V | A | L | S | | | | |
| | | | t | c | | GTG | GcG | tTG | tcG | | | | |
| | | | | | | 0.81 | 0.09 | 0.09 | 0.01 | | | | |
| 147 | A145 | S, P | G | C | C | A | P | S | | | | | |
| | | c, t | | | | GCC | cCC | tCC | | | | | |
| | | | | | | 0.8 | 0.1 | 0.1 | | | | | |

| 149 | T147 | N, K | A | C | C | T | N | K | | | | |
|-----|------|------|---|------|-----|----------|-----|-----|-----|-----|-----|------|
| | | | | a | G | ACC, ACG | AaC | AaG | | | | |
| | | | | | | 0.9 | 0.05 | 0.05 | | | | |
| 167 | K163 | I,T,V | A | A | A | K | I | R | T | E | V | G | A |
| | | | g | t,g,c | | AAA | AtA | AgA | AcA | gAA | gtA | ggA | gcA |
| | | | | | | 0.63 | 0.09 | 0.09 | 0.09 | 0.07 | 0.01 | 0.01 | 0.01 |
| 183 | S179 | Q | T | C | A | S | A | P | T | stop | E | Q | K |
| | | | g,c,a | a | | TCA | gCA | cCA | aCA | TaA | gaA | caA | aaa |
| | | | | | | 0.63 | 0.09 | 0.09 | 0.09 | 0.07 | 0.01 | 0.01 | 0.01 |

| 203 | S197 | T, Y | T | C | C | S | Y | T | N | | | |
| | | | a | a | | TCC | TaC | aCC | aaC | | | |
| | | | | | | 0.81 | 0.09 | 0.09 | 0.01 | | | |
| 223 | R216 | F | C | G | C | R | L | C | F | | | |
| | | | t | t | | CGC | CtC | tGC | ttC | | | |
| | | | | | | 0.81 | 0.09 | 0.09 | 0.01 | | | |

This approach enables the controlled mutagenesis of key residues in the protein molecule, without the disruption of essential residues, that would otherwise lead to the complete loss of fluorescence. Importantly, the method enables selective control of the first, second, and third position of the codon, thereby enabling the selection of conservative mutations if desired.

To identify key residues in the red fluorescent protein, comparisons were made to known favorable mutations in *Aequorea* GFP, and divergences in the sequences between the various species of Anthozoan GFPs, and particularly the red (drFP583) (SEQ. ID. NO. 5 - nucleic acid, & SEQ. ID. NO. 7 - amino acid) and green (dsFP483) (SEQ. ID. NO. 4) fluorescent proteins from *Discosoma striata*.

To maximize the chance of identifying mutations that confer a favorable characteristic, the level of mutagenesis was designed to result in the wild type amino acid being present at each position mutagenized approximately 80% of the time. This approach (often termed soft mutagenesis) helps to avoid the creation of libraries containing mostly non-functional mutants, a situation that can arise if a protein is relatively sensitive to alterations in its amino acid composition.

To ensure that the entire library of mutants was screened, the mutagenesis was completed in a systematic step by step process. This process limited the total diversity in each library to an acceptable value that could be practically screened in mammalian cells via flow cytometry. For example in **Table 3,** the first mutagenic primer has a total diversity of around $1.05 \times 10^6$, compared to the diversity of the entire library which is of the order of $3.42 \times 10^{17}$. Typical commercially available FACS instrumentation have analysis rates of around $2 - 5 \times 10^4$ cells / second, making a realistic analysis of the entire library impractical in a reasonable time frame. By contrast, a screen of a library of about a million cells is relatively easily accomplished, and can, furthermore, be sorted several times over to ensure that the relatively rare, favorable mutations are identified.

III. SCREENING OF LIBRARIES

Once the mutagenic library of red fluorescent mutants has been subcloned into the retroviral expression vector, a library of retroviral plasmids can be produced using standard packaging cell lines, such as PT67 cells. Supernatant from these cells can then be used to infect the mammalian cells in order to express the mutant fluorescent proteins.

Favorable mutants from this step can be identified by FACS analysis based on their improved fluorescence characteristics and increased brightness when expressed in mammalian cells. Typically cells will be selected based on their brightness (fluorescence emission) around 583 nm when excited at around 558 nm.

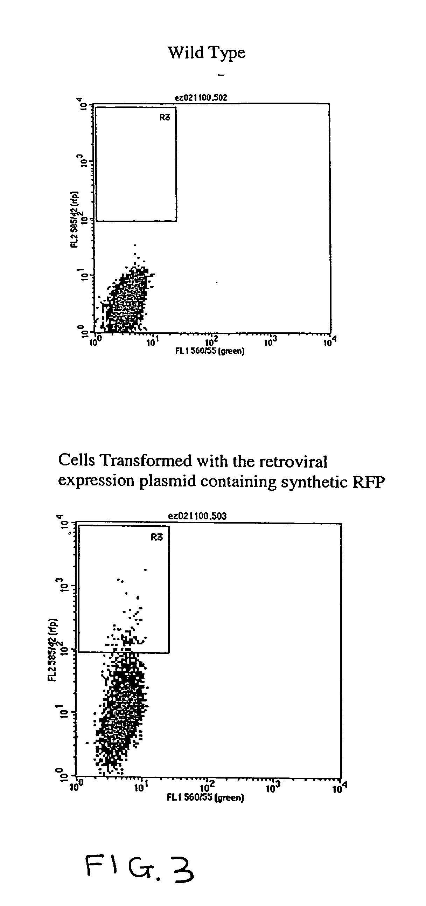

In **FIG. 3,** flow cytometry and cell sorting were conducted using a Becton Dickinson FACSVantage™ SE with a Coherent Innova[R] 70C Spectrum laser producing 60 mW of power at 530.9 nm excitation. The flow cytometer was equipped with pulse processing and the Macrosort™ flow cell. Fluorescence emission was detected via a 585/42 nm bandpass emission filter, separated by a 560 nm short path dichroic mirror. Using the CloneCyt™ Plus integrated deposition system on the FACSVantage™ SE, single cells were sorted into 96-well microtiter plates based on fluorescence intensity (R3) above cellular autofluorescence from a wild type control population. In **FIG. 3,** wild type NIH3T6 cells are shown in the upper panel, while cells transformed with the RFP expression vector ABSC258, are shown in the lower panel. The R3 region represents cells with higher levels of red fluorescence than cellular autofluorescence, and these cells were sorted into 96 well plates for further analysis. In this experiment the sort region (R3) = 0.001 % of the total population in the wild type cells, and 1.40% in the RFP transformed cells.

In addition, multiple rounds of FACS analysis and sorting can be used to selectively enrich mixed pools of brighter mutants to enable the selection of the best mutants. An additional aspect of this strategy is to re-sort the fluorescent cells based on their brightness when excited at 488 nm or 530 nm. In this case one would select for cells with reduced brightness when excited at these wavelengths in order to select for mutants with narrower, sharper excitation peaks.

Another useful sort strategy is to analyze the cells relatively rapidly (i.e. within 24 hours) after transformation in order to identify functional red fluorescent proteins that exhibit more rapid autocatalytic fluorescence development.

Typically after FACS separation, individual cells, or enriched populations of cells, can be sorted into culture plates and allowed to recover for a period of about two weeks. After this period individual cell colonies are typically large enough for further analysis either by further rounds of FACS or via a 96 well plate reader. Analysis via a plate reader provides for accurate quantification and enables a determination of the relative magnitude of the excitation peaks at 487 nm, 530 nm and 558 nm in the same sample. Once colonies expressing mutants with improved characteristics are identified, the sequences of the mutants can be rapidly identified via PCR based sequencing. This can be achived, for example, by using standard fluorescent dye terminator chemistries on a Perkin Elmer 373 or similar automated sequencer, using direct sequencing of PCR products as described by Townley et al., (1997) Genome Res. 7: (3) 293-8. Methods for DNA sequencing are well known in the art and employ such enzymes as the Klenow fragment of DNA polymerase I, SEQUENASE™ (US Biochemical Corp) or Taq polymerase. Methods to extend the DNA from an oligonucleotide primer annealed to the DNA template of interest have been developed for both single- and double-stranded templates. Chain termination reaction products are separated using electrophoresis and detected via their incorporated, labeled precursors.

Recent improvements in mechanized reaction preparation, sequencing and analysis have permitted expansion in the number of sequences that can be determined per day. Preferably, the process is automated with machines such as the Hamilton Micro Lab 2200 (Hamilton, Reno, Nev.), Peltier Thermal Cycler (PTC200; MJ Research, Watertown Mass.) and the Applied Biosystems Catalyst 800 and 377 and 373 DNA sequencers.

The best mutants from this first round of mutagenesis may then be used as the starting product for the next round of mutagenesis. As previously described, oligonucleotides containing a reasonable total diversity are selected to ensure that a complete and thorough search of all of the mutants can be rapidly completed.

After all the mutagenic steps have been completed it is possible to further enhance the fluorescence properties via the use of error prone PCR or Ping pong mutagenesis approaches using methods known in the art to create a highly optimized red fluorescent protein.

Another mutagenesis step may then be completed by recombining the entire pool of favorable mutants to select the most favorable combinations. In this approach the probability of mutagenesis at each position is approximately 50 %, and all the mutations have an equal probability of incorporation into the template fluorescent protein. The most favorable combinations of mutations are then selected to provide for the greatest improvements in brightness and fluorescent properties.


IV. USE AS A MARKER OF GENE EXPRESSION AND CELL MOVEMENT


Typically the functional red fluorescent proteins of the present invention will be introduced and expressed in target cells via the use of standard molecular biology techniques known in the art.

For cell movement studies, expression of the red fluorescent protein will generally be driven via a cell-type specific promoter, in order to be able to selectively monitor the movement of the target cell type. In some cases, for example in cell mixing experiments, it will be preferred for expression to be driven via a constitutive promoter, in other cases it may be preferable to drive expression from an inducible, or developmentally regulated promoter in order to monitor cellular differentiation.

In another embodiment it may be desirable to include additional spectrally resolved fluorescent proteins to simultaneously track both cell movement and differentiation in order to determine both when and where gene expression is modulated. In both cases, nucleic acids in the form of an expression vector including expression control sequences operatively linked to a nucleotide sequence coding for expression of the red fluorescent protein will be used for introducing the proteins into cells. As used, the term "nucleotide sequence coding for expression of" a polypeptide refers to a sequence that, upon transcription and translation of mRNA, produces the polypeptide. This can include sequences containing, *e.g.*, introns. As used herein, the term "expression control sequences" refers to nucleic acid sequences that regulate the expression of a nucleic acid sequence to which it is operatively linked. Expression control sequences are operatively linked to a nucleic acid sequence when the expression control sequences control and regulate the transcription and, as appropriate, translation of the nucleic acid sequence. Thus, expression control sequences can include appropriate promoters, enhancers, transcription terminators, a start codon (*i.e.*, ATG) in front of a protein-encoding gene, splicing signals for introns, IRES sequences (internal ribosome entry site) maintenance of the correct reading frame of that gene to permit proper translation of the mRNA, and stop codons.

Methods that are well known to those skilled in the art can be used to construct expression vectors containing the red fluorescent proteins. These methods include *in vitro* recombinant DNA techniques, synthetic techniques and *in vivo* recombination/genetic recombination. (See, for example, the techniques described in Maniatis, *et al.*,(1989) Cold Spring Harbor Laboratory, N.Y). Many commercially available expression vectors are available from a variety of sources including Clontech (Palo Alto, CA), Stratagene (San Diego, CA) and Invitrogen (San Diego, CA) as well as many other commercial sources.

A contemplated version of the method is to use inducible controlling nucleotide sequences to produce a sudden increase in the expression of the RFP construct e.g., by inducing expression of the construct. Examplary inducible systems include the tetracycline inducible system first described by Bujard and colleagues (Gossen and Bujard (1992) Proc. Natl. Acad. Sci USA 89 5547-5551, Gossen et al. (1995) Science 268 1766-1769) and described in U.S. Patent No 5,464,758.

*Transformation of cells*

Transformation of a host cell with recombinant DNA may be carried out by conventional techniques as are well known to those skilled in the art. Where the host is prokaryotic, such as *E. coli*, competent cells that are capable of DNA uptake can be prepared from cells harvested after exponential growth phase and subsequently treated by the $CaCl_2$ method by procedures well known in the art. Alternatively, $MgCl_2$ or RbCl can be used. Transformation can also be performed after forming a protoplast of the host cell or by electroporation.

When the host is an eukaryote, such methods of transfection of DNA as calcium phosphate co-precipitates, conventional mechanical procedures such as microinjection, electroporation, insertion of a plasmid encased in liposomes, or virus vectors may be used. Eukaryotic cells can also be co-transfected with DNA sequences encoding the fusion polypeptide of the invention, and a second foreign DNA molecule encoding a selectable phenotype, such as the herpes simplex thymidine kinase gene. Another method is to use an eukaryotic viral vector, such as simian virus 40 (SV40) or bovine papilloma virus, to transiently infect or transform eukaryotic cells and express the protein. (Eukaryotic Viral Vectors, Cold Spring Harbor Laboratory, Gluzman ed., 1982). Preferably, an eukaryotic host is utilized as the host cell as described herein.

## V. USE AS A FUSION TAG

The functional red fluorescent proteins of this invention are useful to track the movement of proteins in cells. In this embodiment, a nucleic acid molecule encoding the fluorescent protein is fused in frame to a nucleic acid molecule encoding the protein of interest in an expression vector. Upon expression inside the cell, the protein of interest can be localized based on fluorescence. Typically the protein of interest would be coupled to the RFP via a flexible linker to ensure that both the target protein and fluorescent protein functioned correctly and were efficiently folded. Methods for constructing and introducing such fusion proteins are well known in the art and are also discussed above.

In another version, two or more proteins of interest are simultaneously tracked by fusing the first protein with a functional red fluorescent protein, and the second protein fused to a second fluorescent protein, such as one of the proteins listed in **Table 1.** Typically the second fluorescent protein is chosen based on its fluorescent properties so that it can be spectrally resolved from the functional red fluorescent protein.

## VI. USE IN TRANSGENIC ORGANISMS

In one embodiment, the invention provides a transgenic non-human organism that expresses a nucleic acid sequence that encodes a functional red fluorescent protein. Because such constructs can be expressed within intact living organisms without the need to add co-factors or reagents, and the red emission passes well through tissues, the red fluorescent proteins enable the monitoring of cell movement and differentiation within the entire, intact, living organism.

In another embodiment, the invention can be used to identify where in specific tissues a particular cell type is located, for example, by expression of a red fluorescent protein from a tissue or cell type specific promoter. In another embodiment it may be desirable to include additional spectrally resolved fluorescent proteins to simultaneously track both cell movement and differentiation in order to determine both when and where gene expression is modulated. Such non-human organisms include vertebrates such as rodents, fish such as Zebrafish, non-human primates and reptiles as well as invertebrates. Preferred non-human organisms are selected from the rodent family including rat and mouse, most preferably mouse. The transgenic non-human organisms of the invention are produced by introducing transgenes into the germline of the non-human organism. Embryonic target cells at various developmental stages can be used to introduce transgenes. Different methods are used depending on the organism and stage of development of the embryonic target cell. In vertebrates, the zygote is the best target for microinjection. In the mouse, the male pronucleus reaches the size of approximately 20 micrometers in diameter, which allows reproducible injection of 1-2 p1 of DNA solution. The use of zygotes as a target for gene transfer has a major advantage in that in most cases the injected DNA will be incorporated into the host gene before the first cleavage (Brinster et al., (1985) Proc. Natl. Acad. Sci. USA 82 4438-4442,). As a consequence, all cells of the transgenic non-human animal will carry the incorporated transgene. This will in general also be reflected in the efficient transmission of the transgene to offspring of the founder since 50% of the germ cells will harbor the transgene. Microinjection of zygotes is the preferred method for incorporating transgenes in practicing the invention.

A non-human transgenic organism can be produced by cross-breeding two chimeric organisms which include exogenous genetic material within cells used in reproduction. Twenty-five percent of the resulting offspring will be transgenic *i.e.*, organisms that include the exogenous genetic material within all of their cells in both alleles. 50% of the resulting organisms will include the exogenous genetic material within one allele and 25% will include no exogenous genetic material.

Retroviral infection can also be used to introduce transgene into a non-human organism. In vertebrates, the developing non-human embryo can be cultured *in vitro* to the blastocyst stage. During this time, the blastomeres can be targets for retro viral infection (Jaenich, R., (1976) Proc. Natl. Acad. Sci USA 73 1260-1264,). Efficient infection of the blastomeres is obtained by enzymatic treatment to remove the zona pellucida (Hogan, et al. (1986) in Manipulating the Mouse Embryo, Cold Spring Harbor Laboratory Press, Cold Spring Harbor, N.Y). The viral vector system used to introduce the transgene is typically a replication-defective retrovirus carrying the transgene (Jahner, et al., (1985) Proc. Natl. Acad. Sci. USA 82 6927-6931; Van der Putten, et al., (1985) Proc. Natl. Acad. Sci USA 82 6148-6152). Transfection is easily and efficiently obtained by culturing the blastomeres on a monolayer of virus-producing cells (Van der Putten, *supra*; Stewart, et al., (1987) EMBO J. 6 383-388).

Alternatively, infection can be performed at a later stage. Virus or virus-producing cells can be injected into the blastocoele (D. Jahner et al., (1982) Nature 298 623-628). Most of the founders will be mosaic for the transgene since incorporation occurs only in a subset of the cells that formed the transgenic nonhuman animal. Further, the founder may contain various retro viral insertions of the transgene at different positions in the genome that generally will segregate in the offspring. In addition, it is also possible to introduce transgenes into the germ line, albeit with low efficiency, by intrauterine retroviral infection of the midgestation embryo (D. Jahner *et al.*, *supra*). A third type of target cell for transgene introduction for vertebrates is the embryonic stem cell (ES). ES cells are obtained from pre-implantation embryos cultured *in vitro* and fused with embryos (M. J. Evans et al. (1981) Nature 292 154-156; M.O. Bradley et al., (1984) Nature 309 255-258; Gossler, et al., (1986) Proc. Natl. Acad. Sci USA 83 9065-9069; and Robertson et al., (1986) Nature 322 445-448). Transgenes can be efficiently introduced into the ES cells by DNA transfection or by retro virus-mediated transduction. Such transformed ES cells can thereafter be combined with blastocysts from a nonhuman animal. The ES cells thereafter colonize the embryo and contribute to the germ line of the resulting chimeric animal. (For review see Jaenisch, R., (1988) Science 240 1468-1474).

In another embodiment, the invention provides a transgenic plant that expresses a nucleic acid sequence that encodes red fluorescent protein. Because such constructs can be specifically expressed, both spatially and temporally, within intact living cells, the invention provides the ability to monitor the spatial distribution of a target cell type, within defined cell populations, tissues, or in the entire transgenic plant.

In another embodiment, the approach can be used to specifically identify where in specific tissues a particular gene is expressed, for example by expression of the RFP from tissue specific plant promoters.

In another embodiment it may be desirable to include additional spectrally resolved fluorescent proteins to simultaneously track both cell movement and differentiation in order to determine both when and where gene expression is modulated.

Transgenic plants may be produced by any one of a number of methods of plant transformation and regeneration. Numerous methods for plant transformation have been developed, including biological and physical, plant transformation protocols. See, for example, Miki et al., "Procedures for Introducing Foreign DNA into Plants" in Methods in Plant Molecular Biology and Biotechnology, Glick, B.R. and Thompson, J.E. Eds. (CRC Press, Inc. , Boca Raton, 1993) pages 67-88. In addition, expression vectors and in vitro culture methods for plant cell or tissue transformation and regeneration of plants are available. See, for example, Gruber et al., "Vectors for Plant Transformation" in Methods in Plant Molecular Biology and Biotechnology, Glick, B.R. and Thompson, J.E. Eds. (CRC Press, Inc., Boca Raton, 1993) pages 89-119.

The most widely utilized method for introducing an expression vector into plants is based on the natural transformation system of Agrobacterium. See, for example, Horsch et al., (1985) Science 227 1229. A. tumefaciens and A. rhizogenes are plant pathogenic soil bacteria which genetically transform plant cells. The Ti and Ri plasmids of A. tumefaciens and A. rhizogenes, respectively, carry genes responsible for genetic transformation of the plant See, for example, Kado, C.I., Crit. Rev. Plant. Sci. 10: 1 (1991). Descriptions of Agrobacterium vector systems and methods for Agrobacterium-mediated gene transfer are provided by Gruber et al., supra, Miki et al., supra, and Moloney et al., (1989) Plant Cell Reports 8 238.

Despite the fact the host range for Agrobacterium mediated transformation is broad, some major cereal crop species and gymnosperms have generally been recalcitrant to this mode of gene transfer, even though some success has recently been achieved in rice. Hiei et al., (1994) The Plant Journal 6 271-282. Several methods of plant transformation, collectively referred to as direct gene transfer, have been developed as an alternative to Agrobacterium-mediated transformation.

A generally applicable method of plant transformation is microprojectile-mediated transformation wherein DNA is carried on the surface ofmicroprojectiles measuring 1 to 4 Am. The expression vector is introduced into plant tissues with a biolistic device that accelerates the microprojectiles to speeds of 300 to 600 m/s which is sufficient to penetrate plant cell walls and membranes. Sanford et al., (1987), Part. Sci. Technol. 5 27, Sanford, J.C., (1988) Trends Biotech. 6 299, Sanford, J.C., (1990) Physiol. Plant 79 206, Klein et al., (1992) Biotechnology 10 268.

Another method for physical delivery of DNA to plants is sonication of target cells. Zhang et al., (1991) Bio-Technology 9 996. Alternatively, liposome or spheroplast fusion have been used to introduce expression vectors into plants. Deshayes et al., (1985) EMBO J., 4 2731, Christou et al., (1987) Proc Natl. Acad. Sci. U.S.A. 84 3962. Direct uptakei of DNA into protoplasts using $CaCl_2$ precipitation, polyvinyl alcohol or poly-L-ornithine have also been reported. Hain et al., (1985) Mol.Gen. Genet. 199 161 and Draper et al., (1982) Plant Cell Physiol. 23 451. Electroporation of protoplasts and whole cells and tissues have also been described. Donn et al., In Abstracts of VIIth International Congress on Plant Cell and Tissue Culture IAPTC, A2-38, p 53 (1990) ; D'Halluin et al., (1992) Plant Cell 4 1495-1505 and Spencer et al., (1994) Plant Mol. Biol. 24 51-61.

A preferred method is microprojectile-mediated bombardment of immature embryos. The embryos can be bombarded on the embryo axis side to target the meristem at a very early stage of development or bombarded on the scutellar side to target cells that typically form callus and somatic embryos. Targeting of the scutellum using projectile bombardment is well known to those in the art of cereal tissue culture. Klein et al., (1988) BioTechnol. , 6 559-563; Sautter et al., BiolTechnol., 9 1080-1085 (1991) ; Chibbar et al., (1991) Genome, 34 435-460. The scutellar origin of regenerable callus from cereals is well known. Green et al., (1975) Crop Sci., 15 417-421; Lu et al., (1982)TAG 62 109-112; and Thomas and Scott, (1985) J. Plant Physiol. 121 159-169 - Targeting the scutellum and then using chemical selection to recover transgenic plants is well established in cereals. D/Halluin et al., Plant Cell 4: 1495-1505 (1992) ; Perl et al., MGG 235: 279-284 (1992); Cristou et al., BiolTechnol. 9: 957-962 (1991).

VII. USE FOR FLUORESCENT RESONANCE ENERGY TRANSFER (FRET)

FRET is a general, non-destructive, spectroscopic effect that occurs under certain circumstances (see below) when two fluorophores (a donor fluorophore and acceptor fluorophore) approach closer than about 100 Å. The efficiency of FRET between the two fluorophores is highly distant dependent, and this fact can be exploited to monitor the dynamic association of the fluorophores, or two fluorophore tagged macromolecules. By monitoring FRET between one or more fluorescent proteins it is possible to develop sensitive, non-invasive, cell based assays for a range of activities including proteolysis (see U.S. Patent No. 5,981,200 issued November 9, 1999), analyte determinations (see U.S. Patent No. 5,998,204 issued December 7, 1999) and protein - protein interactions. FRET is most readily determined by measuring the relative emissions of the donor and acceptor fluorophore and then by calculating the emission ratio of these two values. A high degree of FRET is indicated by a high value of the ratio of [acceptor emission/donor emission], and a low degree of FRET is indicated by a low value of this ratio. FRET may also may determined by measuring the degree of donor fluorescence quenching, a measurement method that has the important advantage over emission ratioing in that this value is independent of the concentration the acceptor.

The efficiency of FRET is dependent on the separation distance, the orientation of the donor and acceptor moieties, the fluorescent quantum yield of the donor moiety and the energetic overlap with the acceptor moiety. Forster derived the relationship:

$$E = (F^0 - F)/F^0 = R_0^6/(R^6 + R_0^6)$$

where E is the efficiency of FRET, $F$ and $F^0$ are the fluorescence intensities of the donor in the presence and absence of the acceptor, respectively, and $R$ is the distance between the donor and the acceptor. $R_0$, the distance at which the energy transfer efficiency is 50%, is given (in Å) by

$$R_0 = 9.79 \times 10^3 (K^2 QJn^{-4})^{1/6}$$

where $K^2$ is an orientation factor having an average value close to 0.67 for freely mobile donors and acceptors, $Q$ is the quantum yield of the unquenched fluorescent donor, $n$ is the refractive index of the intervening medium, and $J$ is the overlap integral, which expresses in quantitative terms the degree of spectral overlap,

$$J = \int_{0}^{\infty} {}_{-1}F_{1}\lambda^{4}d\lambda / \int_{0}^{\infty} F_{1}d\lambda$$

where $_{-1}$ is the molar absorptivity of the acceptor in $M^{-1}$ $cm^{-1}$ and $F_{1}$ is the donor fluorescence at wavelength $\lambda$ measured in cm. The dependence of fluorescence energy transfer on the above parameters has been reported [Forster, T. (1948) Ann.Physik 2: 55-75; Lakowicz, J.R., Principles of Fluorescence Spectroscopy, New York:Plenum Press (1983); Herman, B., Resonance energy transfer microscopy, in: Fluorescence Microscopy of Living Cells in Culture, Part B, Methods in Cell Biology, Vol 30, ed. Taylor, D.L. & Wang, Y.-L., San Diego: Academic Press (1989), pp. 219-243; Turro, N.J., Modem Molecular Photochemistry, Menlo Part: Benjamin/Cummings Publishing Co., Inc. (1978), pp. 296-361], and tables of spectral overlap integrals are readily available to those working in the field [for example, Berlman, I.B. Energy transfer parameters of aromatic compounds, Academic Press, New York and London (1973)].

Accordingly, the functional red fluorescent proteins of the present invention are intended to have improved brightness, reduced spectral cross talk and to be rapidly and efficiently expressed in mammalian cells, compared to wild-type Anthozoan proteins. Specifically such proteins are designed to exhibit reduced excitation in the region 400 nm to 515 nm, where most *Aequorea* related donor fluorescent proteins are most efficiently excited, and exhibit an improved molar extinction coefficient when expressed in mammalian cells. Accordingly such functional red fluorescent proteins are useful in any methods that involve FRET.

In one embodiment the functional red fluorescent proteins are useful in FRET based assays for detecting protease activity in which the donor and acceptor fluorescent proteins are separated by a cleavable linker. In this embodiment a first fluorescent protein, for example one of the proteins in **Table 1** is selected as the FRET donor. To optimize the efficiency and detectability of FRET within the tandem fluorescent protein construct, several factors need to be balanced. The emission spectrum of the donor moiety should overlap as much as possible with the excitation spectrum of the acceptor moiety to maximize the overlap integral J. Also, the quantum yield of the donor moiety and the extinction coefficient of the acceptor should likewise be as high as possible to maximize $R_{0}$. However, the excitation spectra of the donor and acceptor moieties should overlap as little as possible so that a wavelength region can be found at which the donor can be excited efficiently without directly exciting the acceptor. Fluorescence arising from direct excitation of the acceptor is difficult to distinguish from fluorescence arising from FRET. Similarly, the emission spectra of the donor and acceptor moieties should overlap as little as possible so that the two emissions can be clearly distinguished. High fluorescence quantum yield of the acceptor moiety is desirable if the emission from the acceptor is to be measured either as the sole readout or as part of an emission ratio. In a preferred embodiment, the donor moiety is typically excited by blue light (<500 nm) and typically emits green light (>500 nm), whereas the acceptor is efficiently excited by green, but not by blue light, and emits red light (>550 nm), for example, preferred donors include Sapphire, W1C, W1B, Emerald. Topaz is preferred for functional red fluorescent proteins that exhibit little or no direct excitation around 500 to 520 nm.

For use in measuring protease activity, the donor and acceptor fluorescent protein moieties are connected through a linker moiety. The linker moiety is preferably a peptide moiety, but can be another organic molecular moiety as well. In a preferred embodiment, the linker moiety includes a cleavage recognition site specific for an enzyme or other cleavage agent of interest. A cleavage site in the linker moiety is useful because when a tandem construct is mixed with the cleavage agent, the linker is a substrate for cleavage by the cleavage agent. Rupture of the linker moiety results in separation of the fluorescent protein moieties that is measurable as a change in FRET.

When the cleavage agent of interest is a protease, the linker can comprise a peptide containing a cleavage recognition motif for the protease. A recognition motif for a protease is a specific amino acid sequence recognized by the protease during proteolytic cleavage. The linker can contain any protease recognition motif known in the art, or discovered in the future.

In one embodiment the functional red fluorescent proteins are useful in FRET based assays for detecting the presence of an analyte (See U.S. Patent No. 5,998,204, issued December 7, 1999). In this case the linker comprising a cleavage site is replaced by a binding protein moiety. The binding protein moiety has an analyte-binding region that binds an analyte and causes the tandem construct to change conformation upon exposure to the analyte. The donor fluorescent protein moiety is covalently coupled to the binding protein moiety. The acceptor fluorescent protein moiety, such as a functional red fluorescent protein, is covalently coupled to the binding protein moiety. In the fluorescent indicator, the donor moiety and the acceptor moiety change position relative to each other when the analyte binds to the analyte-binding region, altering fluorescence resonance energy transfer between the donor moiety and the acceptor moiety when the donor moiety is excited. The change in FRET provides an indication of the concentration of the analyte in the sample.

In another embodiment the functional red fluorescent proteins are useful for FRET based assays for detecting protein-protein interactions. This approach enables an additional range of post-translational activities to be assayed. In this embodiment, a first protein is typically covalently coupled to donor fluorescent protein (such as a fluorescent protein from **Table 1**), and a second protein is covalently coupled to the acceptor fluorescent protein (such as a functional red fluorescent protein). As previously, the donor and acceptor fluorescent proteins are selected to optimize the degree of FRET. Binding of the first protein to the second protein results in the association of the donor and acceptor fluorescent proteins resulting in an enhancement of the degree of FRET between them. This results in a measurable change in the donor and acceptor emission ratio. This approach thus enables the identification and detection of protein-protein interactions between defined proteins, as well as the ability to detect post-translational modifications that influence these protein-protein interactions.

Examples of suitable interaction domains include protein-protein interaction domains such as SH2, SH3, PDZ, 14-3-3, WW and PTB domains. Other interaction domains are described in for example, the database of interacting proteins available on the web at http://www.doe-mbi.ucla.edu.

To identify and characterize the interaction of two test proteins, the method would typically involve; 1) the creation of a first fusion protein comprising the first test protein coupled to the donor fluorescent protein, and a second fusion protein comprising the second test protein coupled to acceptor fluorescent protein; 2) the introduction of the test protein fusion proteins in combination into test cells, and the donor and acceptor fluorescent proteins (without fusion proteins) into control cells; 3) the measurement of the donor and acceptor emission ratios in the control cells and test cells; and 4) comparison of the emission ratio in the control cells, compared to the emission ratio in the test cells.

If the cells expressing the fusion proteins exhibits an emission ratio with a significantly altered value compared to the control cells containing the fluorescent proteins alone, then the results indicate that the two proteins do interact under the experimental conditions chosen. Conversely, if the emission ratios in the control cells, and in the test cells are approximately the same (after taking into account differences in relative expression of the fluorescent proteins), then the results indicate that the proteins probably don't interact strongly under the test conditions.

The method also enables the detection and characterization of stimuli (such as receptor stimulation) that cause two proteins to alter their degree of interaction. In this case, a cell line is created that expresses the first and second fusion proteins, as described above, comprising interaction domains that exhibit, or are believed to exhibit post-translational regulated interactions. For example, post-translational modification by phosphorylation of serine or threonine residues can modulate 14-3-3 domain interactions, tyrosine phosphorylation can influence SH2 domain interactions, the redox state can influence disulfide bond formation. The cell line is then exposed to a test stimulus to determine whether the stimulus regulates the interaction of the two proteins. If the stimulus does regulate the interaction of the two proteins, then this will result in a modulation of the coupling of the two fluorescent proteins, subsequently resulting in a modulation of the degree of FRET and hence fluorescence emission ratio in the treated cells, compared to the non-treated cells.

The invention is also readily amenable to identifying new protein-protein interactions. For example, where a first protein is known, but the protein(s) with which it interacts are unknown. In this case, a first fusion protein is made between the first protein and the donor fluorescent protein (or acceptor fluorescent protein) and cloned into a suitable expression vector. Second, a library of test proteins, for example isolated from a cDNA expression library, is fused in frame to the acceptor fluorescent protein (or donor fluorescent protein) and subcloned into a second expression vector. Typically the first fusion protein would be then be introduced into a population of test cells and single clones identified that stably expressed the fusion protein. The library of test proteins (typically in the form of expression vectors) would be introduced into the clonal cells, stably expressing the first fusion protein. The resulting transformed cells would then be screened to identify cells with altered FRET compared to the control cells. Suitable clones expressing the fusion proteins with modulated FRET, (i.e., altered emission ratios) may then be identified, isolated and characterized, for example by fluorescence activated cell sorting (FACS™). To confirm that the altered emission ratio was indeed the result of FRET, and not due to alterations in the expression level of the acceptor fluorescent protein, secondary measurements of donor emission quenching in the presence and absence of the acceptor would usually be completed. This could be achieved, for example, by measuring donor emission before and after photobleaching of the acceptor. Those library members that display fusion proteins with larger relative changes in emission ratio may then be identified by the degree to which emission ratio is altered for each library member after exposure to the library of test fusion proteins.

VIII. USE FOR DRUG DISCOVERY

FRET based fluorescence assays are well suited for use with systems and methods that utilize automated and integratable workstations for identifying modulators, and chemicals having useful activity. Such systems are described generally in the art (see, U.S. Patent NOs: 4,000,976 to Kramer et al. (issued January 4, 1977), 5,104,621 to Pfost et al. (issued April 14, 1992), 5,125,748 to Bjornson et al. (issued June 30, 1992), 5,139,744 to Kowalski (issued August 18, 1992), 5,206,568 Bjornson et al. (issued April 27, 1993), 5,350,564 to Mazza et al. (September 27, 1994), 5,589,351 to Harootunian (issued December 31, 1996), and PCT Application Nos: WO 93/20612 to Baxter Deutschland GMBH (published October 14, 1993), WO 96/05488 to McNeil et al. (published February 22, 1996), WO 93/13423 to Agong et al. (published July 8, 1993) and U.S. Patent No. 5,985,214, issued November 16, 1999.

Typically, such a system includes: A) a storage and retrieval module comprising storage locations for storing a plurality of chemicals in solution in addressable chemical wells, a chemical well retriever and having programmable selection and retrieval of the addressable chemical wells and having a storage capacity for at least 100,000 addressable wells, B) a sample distribution module comprising a liquid handler to aspirate or dispense solutions from selected addressable chemical wells, the chemical distribution module having programmable selection of, and aspiration from, the selected addressable chemical wells and programmable dispensation into selected addressable sample wells (including dispensation into arrays of addressable wells with different densities of addressable wells per centimeter squared) or at locations, preferably pre-selected, on a plate, C) a sample transporter to transport the selected addressable chemical wells to the sample distribution module and optionally having programmable control of transport of the selected addressable chemical wells or locations on a plate (including adaptive routing and parallel processing), and D) a reaction module comprising either a reagent dispenser to dispense reagents into the selected addressable sample wells or locations on a plate or a fluorescent detector to detect chemical reactions in the selected addressable sample wells or locations on a plate, and a data processing and integration module.

The storage and retrieval module, the sample distribution module, and the reaction module are integrated and programmably controlled by the data processing and integration module. The storage and retrieval module, the sample distribution module, the sample transporter, the reaction module and the data processing and integration module are operably linked to facilitate rapid processing of the addressable sample wells or locations on a plate. Typically, devices of the invention can process at least 100,000 addressable wells or locations on a plate in 24 hours. This type of system is described in commonly owned U.S. Patent No. 5,985,214, issued November 16, 1999. If desired, each separate module is integrated and programmably controlled to facilitate the rapid processing of liquid samples, as well as being operably linked to facilitate the rapid processing of liquid samples. In one embodiment the system provides for a reaction module that is a fluorescence detector to monitor fluorescence. The fluorescence detector is integrated to other workstations with the data processing and integration module and operably linked with the sample transporter. Preferably, the fluorescence detector is of the type described herein and can be used for epi-fluorescence. Other fluorescence detectors that are compatible with the data processing and integration module and the sample transporter, if operable linkage to the sample transporter is desired can be used as known in the art or developed in the future. For some embodiments of the invention, particularly for plates with 96, 192, 384 and 864 wells per plate, detectors are available for integration into the system. Such detectors are described in U.S. Patent 5,589,351 (Harootunian), U.S. Patent 5,355,215 (Schroeder), and PCT patent application WO 93/13423 (Akong). Alternatively, an entire plate may be "read" using an imager, such as a Molecular Dynamics Fluor-Imager 595 (Sunnyvale, CA). Multi-well platforms having greater than 864 wells, including 3,456 wells, can also be used in the present invention (see, for example, the PCT Application PCT/US98/11061, filed 6/2/98. These higher density well plates require miniaturized assay volumes that necessitate the use of highly sensitivity assays that do not require washing. The present invention provides such assays as described herein.

The screening methods described herein can be made on cells growing in or deposited on solid surfaces. A common technique is to use a microtiter plate well wherein the fluorescence measurements are made by commercially available fluorescent plate readers. One such method is to use cells in Costar 96 well microtiter plates (flat with a clear bottom) and measure fluorescent signal with CytoFluor multiwell plate reader (Perseptive Biosystems, Inc., MA) using two emission wavelengths to record fluorescent emission ratios. In another embodiment, the system comprises a microvolume liquid handling system that uses electrokinetic forces to control the movement of fluids through channels of the system, for example as described in U.S. patent No., 5,800,690 issued September 1, 1998 to Chow et al., European patent application EP 0 810 438 A2 filed May 5 1997, by Pelc et al. and PCT application WO 98/00231 filed 24 June 1997 by Parce et al. These systems use "chip" based analysis systems to provide massively parallel miniaturized analysis. Such systems are preferred systems of spectroscopic measurements in some instances that require miniaturized analysis.

*A method for identifying a chemical, modulator or a therapeutic*

The present invention can also be used for testing a therapeutic for useful therapeutic activity. A therapeutic is identified by contacting a test chemical suspected of having a modulating activity of a biological process or target with a test cell comprising the constructs of the present invention. Typically the cells are located within at least one well of a multi-well platform. The test chemical can be part of a library of test chemicals that is screened for activity, such as biological activity. The library can have individual members that are tested individually or in combination, or the library can be a combination of individual members. Such libraries can have at least two members, preferably greater than about 100 members or greater than about 1,000 members, more preferably greater than about 10,000 members, and most preferably greater than about 100,000 or 1,000,000 members. After appropriate incubation of the sample with the test cell, an inhibitor of protein synthesis may be added and a substrate for the reporter moiety added. At least one optical property (such as fluorescence or absorbance) of the sample is determined and compared to a non-treated control to determine the level of reporter gene expression or activity. If the sample having the test chemical exhibits increased or decreased reporter moiety expression or activity relative to that of the control cell then a candidate modulator has been identified.

The candidate modulator can be further characterized and monitored for structure, potency, toxicology, and pharmacology using well-known methods. The structure of a candidate modulator identified by the invention can be determined or confirmed by methods known in the art, such as mass spectroscopy. For putative modulators stored for extended periods of time, the structure, activity, and potency of the putative modulator can be confirmed.

Depending on the system used to identify a candidate modulator, the candidate modulator will have putative pharmacological activity. For example, if the candidate modulator is found to inhibit a protein tyrosine phosphatase involved, for example in T-cell proliferation *in vitro,* then the candidate modulator would have presumptive pharmacological properties as an immunosuppressant or anti-inflammatory (see, Suthanthiran et al., (1996) Am. J. Kidney Disease, 28 159-172) Such nexuses are known in the art for several disease states, and more are expected to be discovered over time. Based on such nexuses, appropriate confirmatory *in vitro* and *in vivo* models of pharmacological activity, as well as toxicology, can be selected. The assays, and methods of use described herein, enable rapid pharmacological profiling to assess selectivity and specificity, and toxicity. This data can subsequently be used to develop new candidates with improved characteristics.

*Bioavailability and Toxicology of Candidate Modulators*

Once identified, candidate modulators can be evaluated for bioavailability and toxicological effects using known methods (see, Lu, Basic Toxicology, Fundamentals, Target Organs, and Risk Assessment, Hemisphere Publishing Corp., Washington (1985); U.S. Patent Nos: 5,196,313 to Culbreth (issued March 23, 1993) and U.S. Patent No. 5,567,952 to Benet (issued October 22, 1996). For example, toxicology of a candidate modulator can be established by determining *in vitro* toxicity towards a cell line, such as a mammalian i.e. human, cell line. Candidate modulators can be treated with, for example, tissue extracts, such as preparations of liver, such as microsomal preparations, to determine increased or decreased toxicological properties of the chemical after being metabolized by a whole organism. The results of these types of studies are often predictive of toxicological properties of chemicals in animals, such as mammals, including humans.

The toxicological activity can be measured using reporter genes that are activated during toxicological activity or by cell lysis (see WO 98/13353, published 4/2/98). Preferred reporter genes produce a fluorescent or luminescent translational product (such as, for example, a Green Fluorescent Protein (see, for example, U.S. Patent No. 5,625,048 to Tsien et al., issued 4/29/98; U.S. Patent No. 5,777,079 to Tsien et al., issued 7/7/98; WO 96/23810 to Tsien, published 8/8/96; WO 97/28261, published 8/7/97; PCT/US97/12410, filed 7/16/97; PCT/US97/14595, filed 8/15/97)) or a translational product that can produce a fluorescent or luminescent product (such as, for example, beta-lactamase (see, for example, U.S. Patent No. 5,741,657 to Tsien, issued 4/21/98, and WO 96/30540, published 10/3/96)), such as an enzymatic degradation product. Cell lysis can be detected in the present invention as a reduction in a fluorescence signal from at least one photon-producing agent within a cell in the presence of at least one photon reducing agent. Such toxicological determinations can be made using prokaryotic or eukaryotic cells, optionally using toxicological profiling, such as described in PCT/US94/00583, filed 1/21/94 (WO 94/17208), German Patent No 69406772.5-08, issued 11/25/97; EPC 0680517, issued 11/12/94; U.S. Patent No. 5,589,337, issued 12/31/96; EPO 651825, issued 1/14/98; and U.S. Patent No. 5,585,232, issued 12/17/96).

Alternatively, or in addition to these *in vitro* studies, the bioavailability and toxicological properties of a candidate modulator in an animal model, such as mice, rats, rabbits, or monkeys, can be determined using established methods (see, Lu, supra (1985); and Creasey, Drug Disposition in Humans, The Basis of Clinical Pharmacology, Oxford University Press, Oxford (1979), Osweiler, Toxicology, Williams and Wilkins, Baltimore, MD (1995), Yang, Toxicology of Chemical Mixtures; Case Studies, Mechanisms, and Novel Approaches, Academic Press, Inc., San Diego, CA (1994), Burrell et al., Toxicology of the Immune System; A Human Approach, Van Nostrand Reinhld, Co. (1997), Niesink et al., Toxicology; Principles and Applications, CRC Press, Boca Raton, FL (1996)). Depending on the toxicity, target organ, tissue, locus, and presumptive mechanism of the candidate modulator, the skilled artisan would not be burdened to determine appropriate doses, $LD_{50}$ values, routes of administration, and regimes that would be appropriate to determine the toxicological properties of the candidate modulator. In addition to animal models, human clinical trials can be performed following established procedures, such as those set forth by the United States Food and Drug Administration (USFDA) or equivalents of other governments. These toxicity studies provide the basis for determining the therapeutic utility of a candidate modulator *in vivo.*

*Efficacy of Candidate Modulators*

Efficacy of a candidate modulator can be established using several art-recognized methods, such as *in vitro* methods, animal models, or human clinical trials (see, Creasey, supra (1979)). Recognized *in vitro* models exist for several diseases or conditions. For example, the ability of a chemical to extend the life-span of HIV-infected cells *in vitro* is recognized as an acceptable model to identify chemicals expected to be efficacious to treat HIV infection or AIDS (see, Daluge et al., (1995) Antimicro. Agents Chemother. 41 1082-1093). Furthermore, the ability of cyclosporin A (CsA) to prevent proliferation of T-cells *in vitro* has been established as an acceptable model to identify chemicals expected to be efficacious as immunosuppressants (see, Suthanthiran *et al.,* supra, (1996)). For nearly every class of therapeutic, disease, or condition, an acceptable *in vitro* or animal model is available. Such models exist, for example, for gastro-intestinal disorders, cancers, cardiology, neurobiology, and immunology. In addition, these *in vitro* methods can use tissue extracts, such as preparations of liver, such as microsomal preparations, to provide a reliable indication of the effects of metabolism on the candidate modulator. Similarly, acceptable animal models may be used to establish efficacy of chemicals to treat various diseases or conditions. For example, the rabbit knee is an accepted model for testing chemicals for efficacy in treating arthritis (see, Shaw and Lacy, J. (1973) Bone Joint Surg. (Br) 55 197-205. Hydrocortisone, which is approved for use in humans to treat arthritis, is efficacious in this model which confirms the validity of this model (see, McDonough, (1982) Phys. Ther. 62 835-839). When choosing an appropriate model to determine efficacy of a candidate modulator, the skilled artisan can be guided by the state of the art to choose an appropriate model, dose, and route of administration, regime, and endpoint and as such would not be unduly burdened.

In addition to animal models, human clinical trials can be used to determine the efficacy of a candidate modulator in humans. The USFDA, or equivalent governmental agencies, have established procedures for such studies (see, www.fda.gov).

*Selectivity of Candidate Modulators*

The *in vitro* and *in vivo* methods described above also establish the selectivity of a candidate modulator. It is recognized that chemicals can modulate a wide variety of biological processes or be selective. Panels of cells, each containing constructs with varying specificity, based on the red fluorescent proteins of the present invention, can be used to determine the specificity of the candidate modulator. Selective modulators are preferable because they have fewer side effects in the clinical setting. The selectivity of a candidate modulator can be established *in vitro* by testing the toxicity and effect of a candidate modulator on a plurality of cell lines that exhibit a variety of cellular pathways and sensitivities. The data obtained from these *in vitro* toxicity studies can be extended into *in vivo* animal model studies, including human clinical trials, to determine toxicity, efficacy, and selectivity of the candidate modulator suing art recognized methods.

*An identified chemical, modulator, or therapeutic and compositions*

The invention includes compositions, such as novel chemicals, and therapeutics identified by at least one method of the present invention as having activity by the operation of methods, systems or components described herein. Novel chemicals, as used herein, do not include chemicals already publicly known in the art as of the filing date of this application. Typically, a chemical would be identified as having activity from using the invention and then its structure can be revealed from a proprietary database of chemical structures or determined using analytical techniques such as mass spectroscopy.

One embodiment of the invention is a chemical with useful activity, comprising a chemical identified by the method described above. Such compositions include small organic molecules, nucleic acids, peptides and other molecules readily synthesized by techniques available in the art and developed in the future. For example, the following combinatorial compounds are suitable for screening: peptoids (PCT Publication No. WO 91/19735, 26 Dec. 1991), encoded peptides (PCT Publication No. WO 93/20242,14 Oct. 1993), random bio-oligomers (PCT Publication WO 92/00091, 9 Jan. 1992), benzodiazepines (U.S. Patent No. 5,288,514), diversomeres such as hydantoins, benzodiazepines and dipeptides (Hobbs DeWitt, S. et al., (1993) Proc. Nat. Acad. Sci. USA 90 6909-6913), vinylogous polypeptides (Hagihara et al., (1992) J. Amer. Chem. Soc. 114 6568), nonpeptidal peptidomimetics with a Beta-D-Glucose scaffolding (Hirschmann, R. et al., (1992) J. Amer. Chem. Soc. 114 9217-9218), analogous organic syntheses of small compound libraries (Chen, C. et al., (1994) J. Amer. Chem. Soc. 116 2661), oligocarbamates (Cho, C.Y. et al., (1993) Science 261: 1303), and/or peptidyl phosphonates (Campbell, D.A. et al., (1994) J. Org. Chem. 59 658). See, generally, Gordon, E. M. et al., (1994). J. Med Chem. 37 1385.

The present invention also encompasses the identified compositions in a pharmaceutical composition comprising a pharmaceutically acceptable carrier prepared for storage and subsequent administration, which have a pharmaceutically effective amount of the products disclosed above in a pharmaceutically acceptable carrier or diluent. Acceptable carriers or diluents for therapeutic use are well known in the pharmaceutical art, and are described, for example, in Remington's Pharmaceutical Sciences, Mack Publishing Co. (A.R. Gennaro edit. 1985). Preservatives, stabilizers, dyes and even flavoring agents may be provided in the pharmaceutical composition. For example, sodium benzoate, acsorbic acid and esters of p-hydroxybenzoic acid may be added as preservatives. In addition, antioxidants and suspending agents may be used.

The compositions of the present invention may be formulated and used as tablets, capsules or elixirs for oral administration; suppositories for rectal administration; sterile solutions, suspensions for injectable administration; and the like. Injectables can be prepared in conventional forms, either as liquid solutions or suspensions, solid forms suitable for solution or suspension in liquid prior to injection, or as emulsions. Suitable excipients are, for example, water, saline, dextrose, mannitol, lactose, lecithin, albumin, sodium glutamate, cysteine hydrochloride, and the like. In addition, if desired, the injectable pharmaceutical compositions may contain minor amounts of nontoxic auxiliary substances, such as wetting agents, pH buffering agents, and the like. If desired, absorption enhancing preparations (e.g., liposomes) may be utilized.

The pharmaceutically effective amount of the composition required as a dose will depend on the route of administration, the type of animal being treated, and the physical characteristics of the specific animal under consideration. The dose can be tailored to achieve a desired effect, but will depend on such factors as weight, diet, concurrent medication and other factors which those skilled in the medical arts will recognize. In practicing the methods of the invention, the products or compositions can be used alone or in combination with one another or in combination with other therapeutic or diagnostic agents. These products can be utilized *in vivo,* ordinarily in a mammal, preferably in a human, or *in vitro.* In employing them *in vivo*, the products or compositions can be administered to the mammal in a variety of ways, including parenterally, intravenously, subcutaneously, intramuscularly, colonically, rectally, nasally or intraperitoneally, employing a variety of dosage forms. Such methods may also be applied to testing chemical activity *in vivo.*

As will be readily apparent to one skilled in the art, the useful *in vivo* dosage to be administered and the particular mode of administration will vary depending upon the age, weight and mammalian species treated, the particular compounds employed, and the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Alternatively, acceptable *in vitro* studies can be used to establish useful doses and routes of administration of the compositions identified by the present methods using established pharmacological methods.

In non-human animal studies, applications of potential products are commenced at higher dosage levels, with dosage being decreased until the desired effect is no longer achieved or adverse side effects disappear. The dosage for the products of the present invention can range broadly depending upon the desired affects and the therapeutic indication. Typically, dosages may be between about 10 mg/kg and 100 mg/kg body weight, and preferably between about 100 $\mu$g/kg and 10 mg/kg body weight. Administration is preferably oral on a daily basis.

The exact formulation, route of administration and dosage can be chosen by the individual physician in view of the patient's condition. (See e.g., Fingl et al., in The Pharmacological Basis of Therapeutics, 1975). It should be noted that the attending physician would know how to and when to terminate, interrupt, or adjust administration due to toxicity, or to organ dysfunctions. Conversely, the attending physician would also know to adjust treatment to higher levels if the clinical response were not adequate (precluding toxicity). The magnitude of an administered dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Depending on the specific conditions being treated, such agents may be formulated and administered systemically or locally. Techniques for formulation and administration may be found in Remington's Pharmaceutical Sciences, 18th Ed., Mack Publishing Co., Easton, PA (1990). Suitable routes may include oral, rectal, transdermal, vaginal, transmucosal, or intestinal administration; parenteral delivery, including intramuscular, subcutaneous, intramedullary injections, as well as intrathecal, direct intraventricular, intravenous, intraperitoneal, intranasal, or intraocular injections.

For injection, the agents of the invention may be formulated in aqueous solutions, preferably in physiologically compatible buffers such as Hanks' solution, Ringer's solution, or physiological saline buffer. For such transmucosal administration, penetrants appropriate to the barrier to be permeated are used in the formulation. Such penetrants are generally known in the art. Use of pharmaceutically acceptable carriers to formulate the compounds herein disclosed for the practice of the invention into dosages suitable for systemic administration is within the scope of the invention. With proper choice of carrier and suitable manufacturing practice, the compositions of the present invention, in particular, those formulated as solutions, may be administered parenterally, such as by intravenous injection. The compounds can be formulated readily using pharmaceutically acceptable carriers well known in the art into dosages suitable for oral administration. Such carriers enable the compounds of the invention to be formulated as tablets, pills, capsules, liquids, gels, syrups, slurries, suspensions and the like, for oral ingestion by a patient to be treated.

Agents intended to be administered intracellularly may be administered using techniques well known to those of ordinary skill in the art. For example, such agents may be encapsulated into liposomes, then administered as described above. All molecules present in an aqueous solution at the time of liposome formation are incorporated into the aqueous interior. The liposomal contents are both protected from the external micro-environment and, because liposomes fuse with cell membranes, are efficiently delivered into the cell cytoplasm. Additionally, due to their hydrophobicity, small organic molecules may be directly administered intracellularly.

Pharmaceutical compositions suitable for use in the present invention include compositions wherein the active ingredients are contained in an effective amount to achieve its intended purpose. Determination of the effective amounts is well within the capability of those skilled in the art, especially in light of the detailed disclosure provided herein. In addition to the active ingredients, these pharmaceutical compositions may contain suitable pharmaceutically acceptable carriers comprising excipients and auxiliaries which facilitate processing of the active compounds into preparations which can be used pharmaceutically. The preparations formulated for oral administration may be in the form of tablets, dragees, capsules, or solutions. The pharmaceutical compositions of the present invention may be manufactured in a manner that is itself known, for example, by means of conventional mixing, dissolving, granulating, dragee-making, levitating, emulsifying, encapsulating, entrapping, or lyophilizing processes.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions.

Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses. Such formulations can be made using methods known in the art (see, for example, U.S. Patent Nos. 5,733,888 (injectable compositions); 5,726,181 (poorly water soluble compounds); 5,707,641 (therapeutically active proteins or peptides); 5,667,809 (lipophilic agents); 5,576,012 (solubilizing polymeric agents); 5,707,615 (anti-viral formulations); 5,683,676 (particulate medicaments); 5,654,286 (topical formulations); 5,688,529 (oral suspensions); 5,445,829 (extended release formulations); 5,653,987 (liquid formulations); 5,641,515 (controlled release formulations) and 5,601,845 (spheroid formulations).

**Claims**

1. A nucleic acid molecule comprising a nucleotide sequence encoding a functional red fluorescent protein comprising one or more mutations such that its sequence differs from the amino acid sequence of an Anthozoan red fluorescent protein of SEQ ID NO: 7, wherein the functional red fluorescent protein is **characterised by** one or more of the following amino acid substitutions:

   Q64K, Q64P, Q64T, Q64N and Q64R;
   F65L, F65V, F65I, F65M, F65Y and F65W;
   Q66R, Q66P, Q66K, Q66E, Q66T, Q66A and Q66G;
   S69L, S69A, S69V and S69T;
   K70M, K70Q, K70L and K70R;
   V71C, V71 L, V71A and V71I;
   Y72F and Y72W;
   V73A, V73L, V73S and V73I;
   A145P, A145S, A145G and A145L;
   T147N, T147K and T147S;
   K163I, K163R, K163T, K163E, K163V, K163G and K163A;
   S179A, S179P, S179T, S179E, S179Q and S179K;
   S197Y, S197T, S197N and S197A; and
   R216K, R216L, R216C and R216F.

2. The nucleic acid molecule of claim 1, wherein said functional red fluorescent protein exhibits a reduced molar extinction coefficient at 487 nm compared to said Anthozoan red fluorescent protein SEQ. ID. No. 7.

3. The nucleic acid molecule of claim 1, wherein said functional red fluorescent protein exhibits a reduced molar extinction coefficient at 530 nm compared to said Anthozoan red fluorescent protein SEQ. ID. NO. 7.

4. The nucleic acid molecule of claim 1, wherein said functional red fluorescent protein exhibits a higher molar extinction coefficient at 583 nm compared to said Anthozoan red fluorescent protein SEQ. ID. NO. 7.

5. The nucleic acid molecule of claim 1, wherein said functional red fluorescent protein is brighter than said Anthozoan red fluorescent protein SEQ. ID. NO. 7 when excited at 558 nm.

6. The nucleic acid molecule of claim 1, wherein said functional red fluorescent protein is brighter than said Anthozoan red fluorescent protein SEQ. ID. NO. 7 when expressed in a mammalian cell grown at 37°C.

7. The nucleic acid molecule of claim 1, wherein said functional red fluorescent protein exhibits a higher quantum yield compared to said Anthozoan red fluorescent protein SEQ. ID. NO. 7.

8. The nucleic acid molecule of claim 1, wherein said functional red fluorescent protein exhibits a faster rate of autocatalytic formation compared to said Anthozoan red fluorescent protein SEQ. ID. NO. 7.

9. The nucleic acid molecule of any preceding claim, wherein at least one or more amino acid substitutions are selected from group consisting of:

Q64K, Q64P, Q64T; Q64N and Q64R;
F65L, F65V, F651, F65M, F65Y and F65W;
Q66R, Q66P, Q66K, Q66E and Q66G;
S69L, S69A, S69V and S69T;
K70M, K70Q, K70L and K70R;
V71C, V71 L, V71A and V71I;
Y72F and Y72W;
V73A, V73L, V73S and V731;
A145P, A145S, A145G and A145L;
T147N, T147K and T147S;
K163I, K163R, K163T, K163E, K163V, K163G and K163A;
S179A, S179P, S179T, S179E, S179Q and S179K;
S197Y, S197T, S197N and S197A; and
R216K, R216L, R216C and R216F.

10. The nucleic acid molecule of claim 1, which consists of the sequence of SEQ. ID. NO. 8 which is modified by mutation to encode a red fluorescent protein as defined in claim 1.

11. An expression vector, comprising expression control sequences operatively linked to the nucleic acid molecule of claim 1.

12. A recombinant host cell, comprising the nucleic acid molecule encoding a functional red fluorescent protein of claim 1.

13. A functional fluorescent protein comprising an amino acid sequence comprising one or more mutations such that it differs from the amino acid sequence of an Anthozoan red fluorescent protein of SEQ. ID. NO. 7, and is **characterised by** one or more of the following amino acid substitutions:

Q64K, Q64P, Q64T, Q64N and Q64R;
F65L, F65V, F651, F65M, F65Y and F65W;
Q66R, Q66P, Q66K, Q66E, Q66T, Q66A and Q66G;
S69L, S69A, S69V and S69T;
K70M, K70Q, K70L and K70R;
V71C, V71 L, V71A and V71I;
Y72F and Y72W;
V73A, V73L, V73S and V731;
A145P, A145S, A145G and A145L;
T147N, T147K and T147S;

K163I, K163R, K163T, K163E, K163V, K163G and K163A;
S179A, S179P, S179T, S179E, S179Q and S179K;
S197Y, S197T, S197N and S197A; and
R216K, R216L, R216C and R216F.

14. A protein of claim 13 which is as further defined in claim 9.

15. A protein of claim 13 which differs from SEQ. ID. NO. 7 by substitution Q64N.

16. A protein of claim 13 which differs from SEQ. ID. NO. 7 by substitution T147S

17. A protein of claim 13 which differs from SEQ. ID. NO. 7 by substitution V71A.

18. A protein of claim 13 which differs from SEQ. ID. NO. 7 by substitution S179T.

19. A protein of claim 13 which differs from SEQ. ID. NO. 7 by substitution S197A.

20. A protein of any of claims 13 to 19 which further has the protein property as further defined in claim 2, claim 3, claim 4, claim 5, claim 6, claim 7 or claim 8.

21. A method of expressing protein of any of claims 13 to 20 **characterised** that the protein is expressed in a mammalian cell.

22. A fusion protein, comprising a protein of interest operably coupled to the functional red fluorescent protein of any of claims 13 to 20.

23. A transgenic non-human organism, comprising a nucleic acid molecule encoding the functional red fluorescent protein of any of claims 13 to 20.

24. A method for identifying a protein-protein interaction, comprising;

a) providing a population of cells comprising the functional red fluorescent protein of any of claims 13 to 20 wherein said functional red fluorescent protein is operably coupled to a first protein of interest,
b) introducing a library of test proteins of interest operably coupled to a functional green fluorescent protein into said population of cells, wherein said functional green fluorescent protein and said functional red fluorescent protein can undergo fluorescence energy transfer (FRET), and
wherein each member of said population of cells receives on average one member of said library of test proteins of interest operably coupled to said functional green fluorescent protein,
(c) screening said population of cells for FRET between said functional green fluorescent protein and said functional red fluorescent protein, and
(d) comparing the FRET in step c) to the FRET in a control cell in the absence of said library of test proteins of interest operably coupled to said functional green fluorescent protein.

25. A method for identifying a modulator of protein-protein interactions, comprising;

(a) contacting a cell with a test chemical, wherein said cell comprises,

i) the functional red fluorescent protein of any of claims 13 to 20 wherein said functional red fluorescent protein is operably coupled to a first protein of interest, and
ii) a functional green fluorescent protein, wherein said functional green fluorescent protein is operably coupled to a second protein of interest, and wherein said functional green fluorescent protein and said functional red fluorescent protein undergo fluorescence energy transfer (FRET) when said first operably coupled protein of interest and said second operably coupled protein of interest associate,

(b) detecting FRET between said functional green fluorescent protein and said functional red fluorescent protein in the presence of said test chemical, and
(c) comparing the FRET in step b) to the FRET in a control cell in the absence of said test chemical,

the method optionally further comprising the step of contacting said cell with an activator prior to the addition of said test chemical or detecting the viability of said cell.

**Patentansprüche**

1. Nukleinsäure-Molekül, umfassend eine Nukleotid-Sequenz, die für ein funktionelles rotes fluoreszierendes Protein kodiert, das eine oder mehrere Mutation(en) derart umfasst, dass sich seine Sequenz von der Aminosäure-Sequenz des roten fluoreszierenden Proteins eines Anthozoons mit der Seq. ID-Nr. 7 unterscheidet, worin das funktionelle rote fluoreszierende Protein **gekennzeichnet ist durch** eine oder mehrere der folgenden Aminosäure-Substitutionen:

   Q64K, Q64P, Q64T, Q64N und Q64R;
   F65L, F65V, F65I, F65M, F65Y und F65W;
   Q66R, Q66P, Q66K, Q66E, Q66T, Q66A und Q66G;
   S69L, S69A, S69V und S69T;
   K70M, K70Q, K70L und K70R;
   V71C, V71L, V71A und V711;
   Y72F und Y72W;
   V73A, V73L, V73S und V731;
   A145P, A145S, A145G und A145L;
   T147N, T147K und T147S;
   K1631, K163R, K163T, K163E, K163V, K163G und K163A;
   S179A, S179P, S179T, S179E, S179Q und S179K;
   S197Y, S197T, S197N und S197A; und
   R216K, R216L, R216C und R216F.

2. Nukleinsäure-Molekül nach Anspruch 1, worin das funktionelle rote fluoreszierende Protein einen reduzierten molaren Extinktions-Koeffizienten bei 487 nm zeigt, verglichen mit dem roten fluoreszierenden Protein eines Anthozoons mit der Seq. ID-Nr. 7.

3. Nukleinsäure-Molekül nach Anspruch 1, worin das funktionelle rote fluoreszierende Protein einen reduzierten molaren Extinktions-Koeffizienten bei 530 nm zeigt, verglichen mit dem roten fluoreszierenden Protein eines Anthozoons mit der Seq. ID-Nr. 7.

4. Nukleinsäure-Molekül nach Anspruch 1, worin das funktionelle rote fluoreszierende Protein einen höheren molaren Extinktions-Koeffizienten bei 583 nm zeigt, verglichen mit dem roten fluoreszierenden Protein eines Anthozoons mit der Seq. ID-Nr. 7.

5. Nukleinsäure-Molekül nach Anspruch 1, worin das funktionelle rote fluoreszierende Protein heller ist als das rote fluoreszierende Protein eines Anthozoons mit der Seq. ID-Nr. 7, wenn es bei 558 nm angeregt wird.

6. Nukleinsäure-Molekül nach Anspruch 1, worin das funktionelle rote fluoreszierende Protein heller ist als das rote fluoreszierende Protein eines Anthozoons mit der Seq. ID-Nr. 7, wenn es in einer Säugerzelle exprimiert wird, die bei 37 °C gezüchtet wurde.

7. Nukleinsäure-Molekül nach Anspruch 1, worin das funktionelle rote fluoreszierende Protein eine höhere Quantenausbeute zeigt, verglichen mit dem roten fluoreszierenden Protein eines Anthozoons mit der Seq. ID-Nr. 7.

8. Nukleinsäure-Molekül nach Anspruch 1, worin das funktionelle rote fluoreszierende Protein eine schnellere Rate der autokatalytischen Bildung zeigt, verglichen mit dem funktionellen roten fluoreszierenden Protein eines Anthozoons mit der Seq. ID-Nr. 7.

9. Nukleinsäure-Molekül nach irgendeinem vorangehenden Anspruch, worin wenigstens eine oder mehrere Aminosäure-Substitutionen gewählt sind aus der Gruppe, die besteht aus:

   Q64K, Q64P, Q64T, Q64N und Q64R;
   F65L, F65V, F65I, F65M, F65Y und F65W;

38

EP 1 259 608 B1

Q66R, Q66P, Q66K, Q66E, und Q66G;
S69L, S69A, S69V und S69T;
K70M, K70Q, K70L und K70R;
V71C, V71L, V71A und V71I;
Y72F und Y72W;
V73A, V73L, V73S und V73I;
A145P, A145S, A145G und A145L;
T147N, T147K und T147S;
K163I, K163R, K163T, K163E, K163V, K163G und K163A;
S179A, S179P, S179T, S179E, S179Q und S179K;
S197Y, S197T, S197N und S197A; und
R216K, R216L, R216C und R216F.

**10.** Nukleinsäure-Molekül nach Anspruch 1, welches aus der Sequenz der Seq. ID-Nr. 8 besteht, die modifiziert ist durch Mutation zum Kodieren eines roten fluoreszierenden Proteins, wie es in Anspruch 1 definiert ist.

**11.** Expressions-Vektor, umfassend Expressions-Steuerungssequenzen, die operativ mit dem Nukleinsäure-Molekül von Anspruch 1 verbunden sind.

**12.** Rekombinante Wirtszelle, umfassend das Nukleinsäure-Molekül, das für ein funktionelles rotes fluoreszierendes Protein gemäß Anspruch 1 kodiert.

**13.** Funktionelles fluoreszierendes Protein, umfassend eine Aminosäure-Sequenz, die eine oder mehrere Mutation(en) derart umfasst, dass sie sich von der Aminosäure-Sequenz eines roten fluoreszierenden Proteins eines Anthozoons mit der Seq. ID-Nr. 7 unterscheidet und **gekennzeichnet ist durch** eine oder mehrere der folgenden Aminosäure-Substitution(en):

Q64K, Q64P, Q64T, Q64N und Q64R;
F65L, F65V, F65I, F65M, F65Y und F65W;
Q66R, Q66P, Q66K, Q66E, Q66T, Q66A und Q66G;
S69L, S69A, S69V und S69T;
K70M, K70Q, K70L und K70R;
V71C, V71L, V71A und V71I;
Y72F und Y72W;
V73A, V73L, V73S und V73I;
A145P, A145S, A145G und A145L;
T147N, T147K und T147S;
K163I, K163R, K163T, K163E, K163V, K163G und K163A;
S179A, S179P, S179T, S179E, S179Q und S179K;
S197Y, S197T, S197N und S197A; und
R216K, R216L, R216C und R216F.

**14.** Protein nach Anspruch 13, welches so ist, wie es weiter in Anspruch 9 definiert ist.

**15.** Protein nach Anspruch 13, welches sich von Seq. ID-Nr. 7 durch eine Substitution Q64N unterscheidet.

**16.** Protein nach Anspruch 13, welches sich von Seq. ID-Nr. 7 durch eine Substitution T147S unterscheidet.

**17.** Protein nach Anspruch 13, welches sich von Seq. ID-Nr. 7 durch eine Substitution V71A unterscheidet.

**18.** Protein nach Anspruch 13, welches sich von Seq. ID-Nr. 7 durch eine Substitution S 179T unterscheidet.

**19.** Protein nach Anspruch 13, welches sich von Seq. ID-Nr. 7 durch eine Substitution S 197A unterscheidet.

**20.** Protein nach einem der Ansprüche 13 bis 19, welches weiter die Protein-Eigenschaft aufweist, wie sie weiter in Anspruch 2, Anspruch 3, Anspruch 4, Anspruch 5, Anspruch 6, Anspruch 7 oder Anspruch 8 definiert ist.

**21.** Verfahren zum Exprimieren von Protein nach irgendeinem der Ansprüche 13 bis 20, **gekennzeichnet dadurch,**

**dass** das Protein in einer Säugerzelle exprimiert wird.

22. Fusionsprotein, umfassend ein Protein von Interesse, das in Funktion an das funktionelle rote fluoreszierende Protein nach irgendeinem der Ansprüche 13 bis 20 gekoppelt ist.

23. Transgener, nicht-menschlicher Organismus, umfassend ein Nukleinsäure-Molekül, das für das funktionelle rote fluoreszierende Protein nach irgendeinem der Ansprüche 13 bis 20 kodiert.

24. Verfahren zum Identifizieren einer Protein-Protein-Wechselwirkung, umfassend:

a) Bereitstellen einer Population von Zellen, die das funktionelle rote fluoreszierende Protein nach irgendeinem der Ansprüche 13 bis 20 umfassen, worin das funktionelle rote fluoreszierende Protein in Funktion an ein erstes Protein von Interesse gekoppelt ist;

b) Einführen einer Bibliothek von Test-Proteinen von Interesse, die in Funktion an ein funktionelles grünes fluoreszierendes Protein gekoppelt sind, in die Population von Zellen, worin das funktionelle grüne fluoreszierende Protein und das funktionelle rote fluoreszierende Protein eine Fluoreszenz-Energie-Übertragung (fluorescence energy transfer; FRET) eingehen können und worin jedes Mitglied der Population von Zellen im Mittel ein Mitglied der Bibliothek von Test-Proteinen von Interesse empfängt, die in Funktion an das funktionelle grüne fluoreszierende Protein gekoppelt sind,

c) Screenen der Population von Zellen auf einen FRET zwischen dem funktionellen grünen fluoreszierenden Protein und dem funktionellen roten fluoreszierenden Protein; und

d) Vergleichen des FRET in Schritt c) mit den FRET in einer Kontrollzelle in Abwesenheit der Bibliothek von Test-Proteinen von Interesse, die in Funktion an das funktionelle grüne fluoreszierende Protein gekoppelt sind.

25. Verfahren zum Identifizieren eines Modulators von Protein-Protein-Wechselwirkungen; umfassend:

a) In-Kontakt-Bringen einer Zelle mit einer Test-Chemikalie, worin die Zelle umfasst:

i. das funktionelle rote fluoreszierende Protein nach irgendeinem der Ansprüche 13 bis 20, worin das funktionelle rote fluoreszierende Protein in Funktion an ein erstes Protein von Interesse gekoppelt ist; und

ii. ein funktionelles grünes fluoreszierendes Protein, worin das funktionelle grüne fluoreszierende Protein in Funktion an ein zweites Protein von Interesse gekoppelt ist und worin das funktionelle grüne fluoreszierende Protein und das funktionelle rote fluoreszierende Protein eine Fluoreszenz-Energie-Übertragung (fluorescence energy transfer; FRET) eingehen, wenn das erste in Funktion gekoppelte Protein von Interesse und das zweite, in Funktion gekoppelte Protein von Interesse assoziierten,

b) Nachweisen eines FRET zwischen dem funktionellen grünen fluoreszierenden Protein und dem funktionellen roten fluoreszierenden Protein in Gegenwart der Test-Chemikalie; und

c) Vergleichen des FRET in Schritt b) mit dem FRET in einer Kontrollzelle in Abwesenheit der Test-Chemikalie;

wobei das Verfahren gegebenenfalls weiter den Schritt eines In-Kontakt-Bringens der Zelle mit einem Aktivator vor der Zugabe der Test-Chemikalie oder Nachweis der Lebensfähigkeit der Zelle umfasst.

**Revendications**

1. Une molécule d'acide nucléique comportant une séquence de nucléotide codant une protéine fluorescente rouge fonctionnelle comprenant une ou plusieurs mutations de façon que sa séquence diffère de la séquence d'acide aminé d'une protéine fluorescente rouge d'Anthozoaire de SEQ. ID. No. 7, où la protéine fluorescente rouge fonctionnelle est **caractérisée par** une ou plusieurs des substitutions d'acide aminé suivantes:

Q64K, Q64P, Q64T, Q64N et Q64R;
F65L, F65V, F65I, F65M, F65Y et F65W;
Q66R, Q66P, Q66K, Q66E, Q66T, Q66A et Q66G;
S69L, S69A, S69V et S69T;
K70M, K70Q, K70L et K70R;
V71C, V71L, V71A et V71I;
Y72F et Y72W;

V73A, V73L, V73S et V731;
A145P, A145S, A145G et A145L;
T147N, T147K et T147S;
K163I, K163R, K163T, K163E, K163V, K163G et K163A;
S179A, S179P, S179T, S179E, S179Q et S179K;
S197Y, S197T, S197N et S197A; et
R216K, R216L, R216C et R216F.

2. La molécule d'acide nucléique selon la revendication 1, où ladite protéine fluorescente rouge fonctionnelle présente un coefficient d'extinction molaire réduit à 487 nm comparé à ladite protéine fluorescence rouge d'Anthozoaire SEQ. IB. No.7.

3. La molécule d'acide nucléique selon la revendication 1, où ladite protéine fluorescente rouge fonctionnelle présente un coefficient d'extinction molaire réduit à 530 nm comparé à ladite protéine fluorescente rouge d'Anthozoaire SEQ. ID. No. 7.

4. La molécule d'acide nucléique selon la revendication 1, où ladite protéine fluorescente rouge fonctionnelle présente un coefficient d'extinction molaire plus élevé à 583 nm comparé à ladite protéine fluorescente rouge d'Anthozoaire SEQ. ID. No. 7.

5. La molécule d'acide nucléique selon la revendication 1, où ladite protéine fluorescente rouge fonctionnelle est plus lumineuse que ladite protéine fluorescente rouge d'Anthozoaire SEQ. ID. No. 7 quand elle est excitée à 558 nm.

6. La molécule d'acide nucléique selon la revendication 1, où ladite protéine fluorescente rouge fonctionnelle est plus lumineuse que ladite protéine fluorescente rouge d'Anthozoaire SEQ. ID. No. 7 quand elle est exprimée dans une cellule mammifère cultivée à 37°C.

7. La molécule d'acide nucléique selon la revendication 1, où ladite protéine fluorescente rouge fonctionnelle présente un rendement quantique plus élevé comparé à ladite protéine fluorescente rouge d'Anthozoaire SEQ. ID. No.7.

8. La molécule d'acide nucléique selon la revendication 1, où ladite protéine fluorescente rouge fonctionnelle présente une plus grande vitesse de formation autocatalytique comparé à ladite protéine fluorescente rouge d'Anthozoaire SEQ. ID. No. 7.

9. La molécule d'acide nucléique selon l'une quelconque des revendications précédentes, où au moins une ou plusieurs substitutions d'acide aminé sont choisies parmi le groupe comprenant :

Q64K, Q64P, Q64T, Q64N et Q64R;
F65L, F65V, F651, F65M, F65Y et F65W;
Q66R, Q66P, Q66K, Q66E et Q66G;
S69L, S69A, S69V et S69T;
K70M, K70Q, K70L et K70R;
V71C, V71 L, V71 A et V71I,
Y72F et Y72W;
V73A, V73L, V73S et V73I;
A145P, A145S, A145G et A145L;
T147N, T147K et T147S;
K163I, K163R, K163T, K163E, K163V, K163G et K163A;
S179A, S179P, S179T, S179E, S179Q et S179K;
S197Y, S197T, S197N et S197A; et
R216K, R216L, R216C et R216F.

10. La molécule d'acide nucléique selon la revendication 1, qui consiste en la séquence de SEQ. ID. No. 8 modifiée par mutation pour coder une protéine fluorescente rouge comme défini dans la revendication 1.

11. Un vecteur d'expression, comportant des séquences de contrôle d'expression opérationnellement liées à la molécule d'acide nucléique selon la revendication 1.

**12.** Une cellule hôte de recombinaison, comportant la molécule d'acide nucléique codant une protéine fluorescente rouge fonctionnelle selon la revendication 1.

**13.** Une protéine fluorescente fonctionnelle comportant une séquence d'acide aminé comportant une ou plusieurs mutations de façon qu'elle diffère de la séquence d'acide aminé d'une protéine fluorescente rouge d'Anthozoaire SEQ. ID. No. 7, et **caractérisée par** une ou plusieurs des substitutions d'acide aminé suivantes:

Q64K, Q64P, Q64T, Q64N et Q64R;
F65L, F65V, F651, F65M, F65Y et F65W;
Q66R, Q66P, Q66K, Q66E, Q66T, Q66A et Q66G;
S69L, S69A, S69V et S69T;
K70M, K70Q, K70L et K70R;
V71C, V71 L, V71 A et V71I;
Y72F et Y72W;
V73A, V73L, V73S et V73I;
A145P, A145S, A145G et A145L;
T147N, T147K et T147S;
K163I, K163R, K163T, K163E, K163V, K163G et K163A;
S179A, S179P, S179T, S179E, S179Q et S179K;
S197Y, S197T, S197N et S197A; et
R216K, R216L, R216C et R216F.

**14.** Une protéine selon la revendication 13 qui est en outre comme défini dans la revendication 9.

**15.** Une protéine selon la revendication 13 qui diffère de la SEQ. ID. No. 7 par substitution Q64N.

**16.** Une protéine selon la revendication 13 qui diffère de la SEQ. ID. No. 7 par substitution T147S.

**17.** Une protéine selon la revendication 13 qui diffère de la SEQ. ID. No. 7 par substitution V71 A.

**18.** Une protéine selon la revendication 13 qui diffère de la SEQ. ID. No. 7 par substitution S179T.

**19.** Une protéine selon la revendication 13 qui diffère de la SEQ. ID. No. 7 par substitution S197A.

**20.** Une protéine selon l'une quelconque des revendications 13 à 19 qui a en outre la propriété de la protéine telle qu'en outre définie dans la revendication 2, la revendication 3, la revendication 4, la revendication 5, la revendication 6, la revendication 7 ou la revendication 8.

**21.** Un procédé d'expression de la protéine selon l'une quelconque des revendications 13 à 20 **caractérisé en ce que** la protéine est exprimée dans une cellule mammifère.

**22.** Une protéine de fusion, comportant une protéine d'intérêt couplée opérationnellement à la protéine fluorescente rouge fonctionnelle selon l'une quelconque des revendications 13 à 20.

**23.** Un organisme non-humain transgénique, comprenant une molécule d'acide nucléique codant la protéine fluorescente rouge fonctionnelle selon l'une quelconque des revendications 13 à 20.

**24.** Un procédé pour identifier une interaction protéine-protéine, consistant ;

a) à réaliser une population des cellules comportant la protéine fluorescente rouge fonctionnelle selon l'une quelconque des revendications 13 à 20 où ladite protéine fluorescente rouge fonctionnelle est couplée opérationnellement à une première protéine d'intérêt,
b) à introduire une bibliothèque de protéines tests d'intérêt couplées opérationnellement à une protéine fluorescente verte fonctionnelle dans ladite population de cellules, où ladite protéine fluorescente verte fonctionnelle et ladite protéine fluorescente rouge fonctionnelle peuvent subir un transfert d'énergie de fluorescence (FRET), et où chaque élément de ladite population de cellules reçoit en moyenne un élément de ladite bibliothèque de protéines tests d'intérêt couplées opérationnellement à ladite protéine fluorescente verte fonctionnelle,
(c) à examiner ladite population de cellules pour le FRET entre ladite protéine fluorescente verte fonctionnelle et ladite protéine fluorescente rouge fonctionnelle, et

(d) à comparer le FRET de l'étape c) avec le FRET dans une cellule de contrôle en l'absence de ladite bibliothèque de protéines tests d'intérêt couplées opérationnellement à ladite protéine fluorescente verte fonctionnelle.

25. Un procédé d'identification d'un modulateur d'interactions protéine-protéine, consistant :

(a) à mettre en contact une cellule avec un produit chimique test, où ladite cellule comporte,

(i) la protéine fluorescente rouge fonctionnelle selon l'une quelconque des revendications 13 à 20, où ladite protéine fluorescente rouge fonctionnelle est couplée opérationnellement à une première protéine d'intérêt, et

(ii) une protéine fluorescente verte fonctionnelle, où ladite protéine fluorescente verte fonctionnelle est couplée opérationnellement à une seconde protéine d'intérêt, et où ladite protéine fluorescente verte fonctionnelle et ladite protéine fluorescente rouge fonctionnelle subissent un transfert d'énergie de fluorescence (FRET) quand ladite première protéine d'intérêt couplée opérationnellement et ladite seconde protéine d'intérêt couplée opérationnellement s'associent,

(b) à détecter le FRET entre ladite protéine fluorescente verte fonctionnelle et ladite protéine fluorescente rouge fonctionnelle en présence du dit produit chimique test, et

(c) à comparer le FRET selon l'étape b) avec le FRET dans une cellule de contrôle en l'absence du dit produit chimique test,

le procédé comprenant en outre optionnellement l'étape de mise en contact de ladite cellule avec un activateur avant l'addition du dit produit chimique test ou la détection de la viabilité de ladite cellule.

```
                    ApoI
                  wwwwwww
                    EcoRI
                  wwwwwww     PaeR7I
                           wwwwwww
              Tsp509
              wwwww
                    XhoI
                  wwwwwwww
         AcsI                                                      BclI
         wwwwww                                                  wwwwwww
+1                              Met  Val  Arg  Ser  Ser  Lys  Asn  Val  Ile  Lys Glu·
  1   CCGAATTCTC  GAGCCACCAT  GGTGAGGAGC  AGCAAGAACG  TGATCAAGGA
      GGCTTAAGAG  CTCGGTGGTA  CCACTCCTCG  TCGTTCTTGC  ACTAGTTCCT

                                    AviII
                                  wwwwwwwww
           RcaI                     FspI
         wwwwwww                   wwwwwww
           BspHI                    AosI
         wwwwwww                   wwwwwwww
+1   ·Glt Phe Met  Arg  Phe  Lys  Val  Arg  Met  Glu  Gly  Thr  Val  Asn  Gly  His  Glu Phe·
 51   GTTCATGAGG  TTCAAGGTGC  GCATGGAGGG  CACCGTGAAC  GGCCACGAGT
      CAAGTACTCC  AAGTTCCACG  CGTACCTCCC  GTGGCACTTG  CCGGTGCTCA

+1   ·Phe Glu  Ile  Glu  Gly  Glu  Gly  Glu  Gly  Arg  Pro  Tyr  Glu  Gly  His  Asn  Thr
101   TCGAGATCGA  GGGCGAGGGC  GAGGGCAGGC  CCTACGAGGG  CCACAACACC
      AGCTCTAGCT  CCCGCTCCCG  CTCCCGTCCG  GGATGCTCCC  GGTGTTGTGG

              BfrI
            wwwwwwwww
           HindIII
         wwwwwwwww
           AflII                                                  BsmFI
         wwwwwwwww                                              wwwwwww
+1    Val  Lys  Leu  Lys  Val  Thr  Lys  Gly  Gly  Pro  Leu  Pro  Phe  Ala  Trp  Asp  Ile·
151   GTGAAGCTTA  AGGTGACCAA  GGGCGGCCCC  CTGCCCTTCG  CCTGGGACAT
      CACTTCGAAT  TCCACTGGTT  CCCGCCGGGG  GACGGGAAGC  GGACCCTGTA

           BanII
         wwwwwwwww
+1   ·Ile Leu  Ser  Pro  Gln  Phe  Gln  Tyr  Gly  Ser  Lys  Val  Tyr  Val  Lys  His  Pro Ala·
201   CCTGAGCCCC  CAGTTCCAGT  ACGGCAGCAA  GGTGTACGTG  AAGCACCCCG
      GGACTCGGGG  GTCAAGGTCA  TGCCGTCGTT  CCACATGCAC  TTCGTGGGGC

                              CelII
                            wwwwwwwww
                              EspI
                            wwwwwwwww
                              Bpu11021
                            wwwwwwwww
+1   ·Ala Asp  Ile  Pro  Asp  Tyr  Lys  Lys  Leu  Ser  Phe  Pro  Glu  Gly  Phe  Lys  Trp
251   CCGACATCCC  CGACTACAAG  AAGCTGAGCT  TCCCCGAGGG  CTTCAAGTGG
      GGCTGTAGGG  GCTGATGTTC  TTCGACTCGA  AGGGGCTCCC  GAAGTTCACC

+1    Glu  Arg  Val  Met  Asn  Phe  Glu  Asp  Gly  Gly  Val  Val  Thr  Val  Thr  Gln  Asp·
301   GAGAGGGTGA  TGAACTTCGA  GGACGGCGGC  GTGGTGACCG  TGACCCAGGA
      CTCTCCCACT  ACTTGAAGCT  CCTGCCGCCG  CACCACTGGC  ACTGGGTCCT

              SfcI
            wwwwwwwww
              PstI
            wwwwwwwww
+1   ·Asp Ser  Ser  Leu  Gln  Asp  Gly  Cys  Phe  Ile  Tyr  Lys  Val  Lys  Phe  Ile  Gly Val·
351   CAGCAGCCTG  CAGGACGGCT  GCTTCATCTA  CAAGGTGAAG  TTCATCGGCG
      GTCGTCGGAC  GTCCTGCCGA  CGAAGTAGAT  GTTCCACTTC  AAGTAGCCGC

                                              BpuAI
                                            wwwwwwwww
                                SfaNI          BbsI
                              wwwwwww        wwwwwwwww
+1   ·Val Asn  Phe  Pro  Ser  Asp  Gly  Pro  Val  Met  Gln  Lys  Lys  Thr  Met  Gly  Trp
401   TGAACTTCCC  CAGCGACGGC  CCCGTGATGC  AGAAGAAGAC  CATGGGCTGG
      ACTTGAAGGG  GTCGCTGCCG  GGGCACTACG  TCTTCTTCTG  GTACCCGACC
```

FIG. 1

```
                                              FnuDII
                                              ﹏﹏﹏﹏
                                               MvnI
          StuI                                ﹏﹏﹏﹏
       ﹏﹏﹏﹏                                    ThaI
                                              ﹏﹏﹏﹏                        XhoII
       AatI              HaeII                 BstUI                        ﹏
     ﹏﹏﹏﹏             ﹏﹏﹏﹏                ﹏﹏﹏﹏                     BstYI
                                                                             ﹏
 +1  Glu Ala Ser  Thr  Glu Arg Leu  Tyr Pro  Arg Asp Gly  Val  Leu  Lys Gly Glu
451  GAGGCCTCCA CCGAGCGCCT GTACCCCCGC GACGGCGTGC TGAAGGGCGA
     CTCCGGAGGT GGCTCGCGGA CATGGGGGCG CTGCCGCACG ACTTCCCGCT

       BstYI
     ﹏﹏﹏﹏
       XhoII
     ﹏﹏﹏﹏
       AlwI                                               SexAI
     ﹏﹏﹏﹏                                              ﹏﹏﹏﹏﹏
 +1  ·Glu Ile His  Lys Ala Leu Lys  Leu Lys Asp  Gly  Gly His  Tyr Leu Val  Glu Phe·
501  GATCCACAAG GCCCTGAAGC TGAAGGACGG CGGCCACTAC CTGGTGGAGT
     CTAGGTGTTC CGGGACTTCG ACTTCCTGCC GCCGGTGATG GACCACCTCA

              BstXI
       ﹏﹏﹏﹏﹏﹏﹏﹏
              MluNI                                Pvull
            ﹏﹏﹏﹏﹏                               ﹏﹏﹏﹏
              MscI                                        Ncil
            ﹏﹏﹏﹏﹏                          MspA1I  ﹏﹏﹏﹏
               BalI                        ﹏﹏﹏﹏   MspI
            ﹏﹏﹏﹏﹏                         BsgI    ﹏﹏﹏﹏
                                          ﹏﹏﹏﹏   HpaII
 +1  ·Phe Lys Ser  Ile  Tyr Met Ala  Lys Lys Pro  Val Gln Leu  Pro Gly  Tyr Tyr
551  TCAAGTCCAT CTACATGGCC AAGAAGCCCG TGCAGCTGCC CGGCTACTAC
     AGTTCAGGTA GATGTACCGG TTCTTCGGGC ACGTCGACGG GCCGATGATG

 +1  Tyr Val Asp  Ser  Lys Leu Asp  Ile Thr Ser  His Asn Glu  Asp  Tyr Thr Ile·
601  TACGTGGACT CCAAGCTGGA CATCACCAGC CACAACGAGG ACTACACCAT
     ATGCACCTGA GGTTCGACCT GTAGTGGTCG GTGTTGCTCC TGATGTGGTA

                                                                        SalI
                         AvaII                                          ﹏
 +1  ·Ile Val Glu  Gln  Tyr Glu Arg  Thr  Glu Gly  Arg His His  Leu  Phe Leu  ···  AccI
                       ﹏﹏﹏﹏                                                        ﹏
651  CGTGGAGCAG TACGAGAGGA CCGAGGGCAG GCACCACCTG TTCCTGTGAG
     GCACCTCGTC ATGCTCTCCT GGCTCCCGTC CGTGGTGGAC AAGGACACTC

              HpaI
            ﹏﹏﹏﹏
       SalI
     ﹏﹏﹏﹏
       AccI
     ﹏﹏﹏﹏
701  TCGACGTTAA CCC
     AGCTGCAATT GGG
```

FIG. 1 continued

45

FIG. 2

Wild Type

Cells Transformed with the retroviral
expression plasmid containing synthetic RFP

FIG. 3

**REFERENCES CITED IN THE DESCRIPTION**

**Patent documents cited in the description**

- US 5625048 A, Tsien
- US 5777079 A, Tsien
- US 5804387 A, Cormack
- US 5532130 A
- EP 0839830 A
- EP 0742287 A
- EP 0285057 A
- EP 0694559 A
- US 5795737 A
- US 5464758 A
- US 5981200 A
- US 5998204 A
- US 4000976 A, Kramer
- US 5104621 A, Pfost
- US 5125748 A, Bjornson
- US 5139744 A, Kowalski
- US 5206568 A, Bjornson
- US 5350564 A, Mazza
- US 5589351 A, Harootunian
- WO 9320612 A
- WO 9605488 A, McNeil
- WO 9313423 A, Agong
- US 5985214 A
- US 5355215 A, Schroeder
- US 9811061 W
- US 5800690 A, Chow
- EP 0810438 A2, Pelc
- WO 9800231 A, Parce
- US 5196313 A, Culbreth
- US 5567952 A, Benet
- WO 9813353 A
- WO 9623810 A, Tsien
- WO 9728261 A
- US 9712410 W
- US 9714595 W
- US 5741657 A, Tsien
- WO 9630540 A
- US 9400583 W
- WO 9417208 A
- DE 69406772508
- EP C0680517 A
- US 5589337 A
- EP O651825 A
- EP 11498 A
- US 5585232 A
- WO 9119735 A
- WO 9320242 A
- WO 9200091 A
- US 5288514 A
- US 5733888 A
- US 5726181 A
- US 5707641 A
- US 5667809 A
- US 5576012 A
- US 5707615 A
- US 5683676 A
- US 5654286 A
- US 5688529 A
- US 5445829 A
- US 5653987 A
- US 5641515 A
- US 5601845 A

**Non-patent literature cited in the description**

- **Tsien.** *Annu. Rev. Biochem.,* 1998, vol. 67, 509-544
- **Ward ; Bokman.** *Biochemistry,* 1982, vol. 21, 4535-4540
- **Ward et al.** *Photochem. Photobiol.,* 1982, vol. 35, 803-808
- **A. B. Cubitt et al.** *Trends Biochem. Sci.,* 1995, vol. 20, 448-455
- **Shimomura ; Johnson.** *J. Cell. Comp. Physio.,* 1962, vol. 59, 223
- **Cody et al.** *Biochemistry,* 1993, vol. 32, 1212-1218
- **Heim et al.** *Proc. Natl. Acad. Sci. USA,* 1994, vol. 91, 12501-12504
- **Heim et al.** *Nature,* 1995, vol. 373, 664-665
- **D.C. Prasher et al.** *Gene,* 1992, vol. 111, 229-33
- Green Fluorescent Proteins. Academic Press, 19-47
- **Ormo et al.** *Science,* 1996, vol. 273, 1392-1395
- **Yang et al.** *Nat. Biotechnol.,* 1996, vol. 14, 1246-1251
- **Brejc et al.** *Proc. Natl. Acad. Sci. USA,* 1997, vol. 94, 2306-2311
- **Scharnagl et al.** *Biophys J.,* 1999, vol. 77, 1839-1857

- **Elsliger et al.** *Biochem.,* 1999, vol. 38, 5296-5301
- **Matz et al.** *Nature Biotech.,* 1999, vol. 17, 969-973
- **Lakowicz, J.R.** Topics in Fluorescence Spectroscopy. Plenum Press, 1991, vol. 3
- **Lakowicz, J. R.** *Scanning Microsc SuppL,* 1996, vol. 10, 213-24
- **Sambrook et al.** Molecular Cloning: A Laboratory Manual. Cold Spring Harbor Laboratory Press, 1989
- Cells: A Laboratory Manual. Cold Spring Harbor Laboratory Press, 1998
- **Snyder ; Love.** Optical Waveguide Theory. Chapman & Hall
- **Dayhoff.** Atlas of Protein Sequence and Structure. *National Biomedical Research Foundation,* vol. 5, 101-110
- *NATIONAL BIOMEDICAL RESEARCH FOUNDATION,* vol. 2, 1-10
- Proteins- Structure and Molecular Properties. W.H. Freeman and Company, 1993
- Posttranslational Covalent Modification of Proteins. Academic Pres, 1983, 1-12
- **Smith ; Waterman.** *Adv. Appl. Math.,* 1981, vol. 2, 482
- **Needleman ; Wunsch.** *J. Mol. Biol.,* 1970, vol. 48, 443
- **Lipman.** *Proc. Natl. Acad. Sci. (U.S.A.),* 1988, vol. 85, 2444
- **Townley et al.** *Genome Res.,* 1997, vol. 7 (3), 293-8
- **Gossen ; Bujard.** *Proc. Natl. Acad. Sci USA,* 1992, vol. 89, 5547-5551
- **Gossen et al.** *Science,* 1995, vol. 268, 1766-1769
- Eukaryotic Viral Vectors. Cold Spring Harbor Laboratory, 1982
- **Brinster et al.** *Proc. Natl. Acad. Sci. USA,* 1985, vol. 82, 4438-4442
- **Jaenich, R.** *Proc. Natl. Acad. Sci USA,* 1976, vol. 73, 1260-1264
- **Hogan et al.** *Manipulating the Mouse Embryo,* 1986
- **Jahner et al.** *Proc. Natl. Acad. Sci. USA,* 1985, vol. 82, 6927-6931
- **Van der Putten et al.** *Proc. Natl. Acad. Sci USA,* 1985, vol. 82, 6148-6152
- **Stewart et al.** *EMBO J.,* 1987, vol. 6, 383-388
- **D. Jahner et al.** *Nature,* 1982, vol. 298, 623-628
- **M. J. Evans et al.** *Nature,* 1981, vol. 292, 154-156
- **M.O. Bradley et al.** *Nature,* 1984, vol. 309, 255-258
- **Gossler et al.** *Proc. Natl. Acad. Sci USA,* 1986, vol. 83, 9065-9069
- **Robertson et al.** *Nature,* 1986, vol. 322, 445-448
- **Jaenisch, R.** *Science,* 1988, vol. 240, 1468-1474
- Procedures for Introducing Foreign DNA into Plants. **Miki et al.** Methods in Plant Molecular Biology and Biotechnology. CRC Press, Inc, 1993, 67-88
- Vectors for Plant Transformation. **Gruber et al.** Methods in Plant Molecular Biology and Biotechnology. CRC Press, Inc, 1993, 89-119
- **Horsch et al.** *Science,* 1985, vol. 227, 1229
- **Kado, C.I.** *Crit. Rev. Plant. Sci.,* 1991, vol. 10, 1
- **Moloney et al.** *Plant Cell Reports,* 1989, vol. 8, 238
- **Hiei et al.** *The Plant Journal,* 1994, vol. 6, 271-282
- **Sanford et al.** *Part. Sci. Technol.,* 1987, vol. 5, 27
- **Sanford, J.C.** *Trends Biotech.,* 1988, vol. 6, 299
- **Sanford, J.C.** *Physiol. Plant,* 1990, vol. 79, 206
- **Klein et al.** *Biotechnology,* 1992, vol. 10, 268
- **Zhang et al.** *BioTechnology,* 1991, vol. 9, 996
- **Deshayes et al.** *EMBO J.,* 1985, vol. 4, 2731
- **Christou et al.** *Proc Natl. Acad. Sci. U.S.A.,* 1987, vol. 84, 3962
- **Hain et al.** *Mol.Gen. Genet.,* 1985, vol. 199, 161
- **Draper et al.** *Plant Cell Physiol.,* 1982, vol. 23, 451
- **Donn et al.** *Abstracts of VIIth International Congress on Plant Cell and Tissue Culture IAPTC,* 1990, vol. A2-38, 53
- **D'Halluin et al.** *Plant Cell,* 1992, vol. 4, 1495-1505
- **Spencer et al.** *Plant Mol. Biol.,* 1994, vol. 24, 51-61
- **Klein et al.** *BioTechnol,* 1988, vol. 6, 559-563
- **Sautter et al.** *BiolTechnol.,* 1991, vol. 9, 1080-1085
- **Chibbar et al.** *Genome,* 1991, vol. 34, 435-460
- **Green et al.** *Crop Sci.,* 1975, vol. 15, 417-421
- **Lu et al.** *TAG,* 1982, vol. 62, 109-112
- **Thomas ; Scott.** *J. Plant Physiol.,* 1985, vol. 121, 159-169
- **D/Halluin et al.** *Plant Cell,* 1992, vol. 4, 1495-1505
- **Perl et al.** *MGG,* 1992, vol. 235, 279-284
- **Cristou et al.** *BiolTechnol,* 1991, vol. 9, 957-962
- **Forster, T.** *Ann.Physik,* 1948, vol. 2, 55-75
- **Lakowicz, J.R.** Principles of Fluorescence Spectroscopy. Plenum Press

- Resonance energy transfer microscopy, in: Fluorescence Microscopy of Living Cells in Culture. **Herman, B.** Methods in Cell Biology. Academic Press, 1989, vol. 30, 219-243
- **Turro, N.J.** Modem Molecular Photochemistry. Benjamin/Cummings Publishing Co., Inc, 1978, 296-361
- **Berlman, I.B.** Energy transfer parameters of aromatic compounds. Academic Press, 1973
- **Suthanthiran et al.** *Am. J. Kidney Disease,* 1996, vol. 28, 159-172
- **Lu.** *Basic Toxicology, Fundamentals, Target Organs, and Risk Assessment,* 1985
- Drug Disposition in Humans. **Creasey.** The Basis of Clinical Pharmacology. Oxford University Press, 1979
- **Osweiler.** Toxicology. Williams and Wilkins, 1995
- Toxicology of Chemical Mixtures. **Yang.** Case Studies, Mechanisms, and Novel Approaches. Academic Press, Inc, 1994
- Toxicology of the Immune System. **Burrell et al.** A Human Approach. Van Nostrand Reinhld, Co, 1997
- **Niesink et al.** Toxicology; Principles and Applications. CRC Press, 1996
- **Daluge et al.** *Antimicro. Agents Chemother,* 1995, vol. 41, 1082-1093
- **Shaw ; Lacy, J.** *Bone Joint Surg. (Br),* 1973, vol. 55, 197-205
- **McDonough.** *Phys. Ther.,* 1982, vol. 62, 835-839
- **Hobbs DeWitt, S. et al.** *Proc. Nat. Acad. Sci. USA,* 1993, vol. 90, 6909-6913
- **Hagihara et al.** *J. Amer. Chem. Soc.,* 1992, vol. 114, 6568
- **Hirschmann, R. et al.** *J. Amer. Chem. Soc.,* 1992, vol. 114, 9217-9218
- **Chen, C. et al.** *J. Amer. Chem. Soc.,* 1994, vol. 116, 2661
- **Cho, C.Y. et al.** *Science,* 1993, vol. 261, 1303
- **Campbell, D.A. et al.** *J. Org. Chem.,* 1994, vol. 59, 658
- **Gordon, E. M. et al.** *J. Med Chem.,* 1994, vol. 37, 1385
- Remington's Pharmaceutical Sciences. Mack Publishing Co, 1985
- **Fingl et al.** *The Pharmacological Basis of Therapeutics,* 1975
- Remington's Pharmaceutical Sciences. Mack Publishing Co, 1990